# EUROPEAN PATENT SPECIFICATION

(11) **EP 2 380 896 B1**
(45) Date of publication and mention of the grant of the patent: **13.02.2013**
(21) Application number: 09817240.6
(22) Date of filing: 22.09.2009
(51) Int. Cl.: C07H 21/00, C07H 19/167, C07H 19/20, C07H 19/067, C07H 19/10

(54) **A METHOD FOR PREPARING OLIGONUCLEOTIDE**
VERFAHREN ZUR HERSTELLUNG EINES OLIGONUKLEOTIDS
PROCÉDÉ POUR PRÉPARER UN OLIGONUCLÉOTIDE

(30) Priority: 23.09.2008 CN 200810149372
(43) Date of publication of application: 26.10.2011
(73) Proprietor: Suzhou Ribo Life Science Co., Ltd, Jiangsu 215347 (CN)
(72) Inventor: XI, Zhen, Tianjin 300071 (CN); HUANG, Jinyu, Tianjin 300071 (CN); ZHANG, Junbin, Tianjin 300071 (CN)
(74) Representative: Björk, Frida Magdalena
(86) International application number: PCT/CN2009/074101
(87) International publication number: WO 2010/037326

(56) References cited:
- CN-A- 1 245 809
- CN-A- 1 409 719
- CN-A- 101 133 073
- DD-A1- 273 064

## Description

### Field of the Invention

The present invention relates to a method for preparing oligonucleotide.

### Background of the Invention

The basic structural unit of oligonucleotide is nucleotide which comprises base, pentose and phosphoric acid. Nucleotides may be classified into RNA (ribonucleic acid) and DNA (deoxyribonucleic acid) which have the same basic chemical structure, except that they contain different pentose in which the pentose of RNA is D-ribose, and the pentose of DNA is D-2-deoxyribose. The base generally includes guanine, adenine, cytosine, thymine and uracil. The chemical synthesis method of oligonucleotides currently widely adopted is solid phase synthesis method (also known as phosphoramidite-triester method). At pages 520-521 of the Biochemistry (Book 1, Edition 3, Wang Jingyan et al, Higher Education Press), the principle of solid phase synthesis is introduced as follows: All reactions take place in a solid phase column which is no big. Firstly, some free groups on the nucleotides to be activated are protected (blocked) to make the reactions go towards the designed direction. 5'-OH is protected with DMT (4,4'-dimethoxytriphenylmethyl) group, and the amino groups on the base are protected with benzoyl groups. 3'-OH is activated with amino phosphorous acid compound. The 3'-OH of the first nucleotide is combined with the solid phase (resin). A phosphite triester is formed between its 5'-OH and the activated monomer (nucleotide). As the 5'-OH on the activated monomer and the amino group on the base are protected, they won't participate in the reaction. During the reaction in the second step, phosphite triester is oxidized by iodine into phosphate triester; during the reaction in the third step, trichloroacetic acid is added to remove protective agent DMT on 5'-OH in the growing chain. By now, DNA chain has been extended by one more nucleotide unit and is ready for the reactions for next round of extension. According to the program input in advance, after the synthesis of a whole DNA fragment is completed, thiophenol is used to remove the protective agent DMT on 5'-OH, and concentrated ammonium hydroxide is used to disconnect DNA fragment from the solid phase resin so that DNA is eluted. Then concentrated ammonium hydroxide is used again to remove the protective agent on the base under heated condition. Finally ammonium hydroxide is removed under vacuum.

As the whole process of the foregoing solid phase synthesis is conducted in a solid phase column, due to the limitation of the volume of the solid phase column, this method has a small synthesis scale and low yield and can't meet the requirement of mass synthesis. Following the deepening of the research on nucleic acid, on the one hand, the amount of the oligomeric RNA used in scientific research increases rapidly, and on the other hand, the clinical application of RNA is in the ascendant, so it is a matter of significance to design a method for synthesizing RNA on a large scale.

### Summary of the Invention

The object of the present invention is to overcome the shortcoming of the existing chemical synthesis method of oligonucleotides - small synthesis scale and provide a method to synthesize oligonucleotide on a large scale.

The present invention provides a method for preparing oligonucleotide, including reacting the compound represented by Formula (1) with the compound represented by Formula (2) in a liquid reaction medium under the condition of condensation reaction to obtain the compound represented by Formula (3), wherein said condition of condensation reaction includes the use of 1-(mesitylene-2-sulfonyl)-3-nitro-1,2,4-triazole (MSNT) as condensing agent,

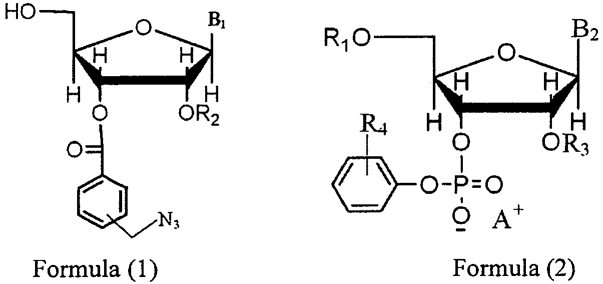

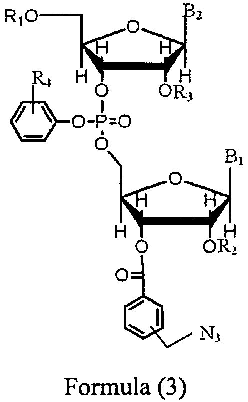

Wherein:
R₁ represents 4,4'-dimethoxytriphenylmethyl, RNA or DNA;
R₂ and R₃ independently represent a sterically hindered silane protective group;
R₄ represents a halogen atom;
A⁺ represents a tri-alkyl ammonium ion;
B₁ and B₂ independently represent guanyl substituted with N-acyl, adeninyl substituted with N-acyl, cytosine substituted with N-acyl, thymine or uracil.

The method provided by the present invention adopts appropriate protective groups to protect corresponding functional groups, such that only the 5'-OH to which the compound represented by Formula (1) needs to be connected and the 3'-phosphate to which the compound represented by Formula (2) needs to be connected are exposed. The condensation reaction takes place in the liquid reaction medium to connect OH-component and P-component and obtain DNA or RNA short chains. If necessary, after this step is completed, the 5'-protective groups of the newly obtained DNA or RNA short chains may be removed, condensation reaction with the new compound represented by Formula (2) may take place again to obtain longer chains, the reaction is repeated several times and finally all protective groups are removed such that the oligonucleotides with the needed length are obtained; alternatively, the 3'-protective group of the newly obtained DNA or RNA short chains may be removed, then 3'-hydroxyl phosphate is esterified to generate a new P-component (the reaction shown in Reaction Scheme III), it again takes condensation reaction with the new compound represented by Formula (3) to obtain longer chains, the reaction is repeated several times and finally all protective groups are removed such that the expected oligonucleotides are obtained. The method provided by the present invention doesn't need a solid phase column and the reactions may take place in a liquid reaction medium, so oligonucleotides can be synthesized on a large scale.

### Detailed Description of the Embodiments

The method for preparing oligonucleotide according to the present invention includes reacting the compound represented by Formula (1) with the compound represented by Formula (2) in a liquid reaction medium under the condition of condensation reaction to obtain the compound represented by Formula (3), wherein said condition of condensation reaction includes the use of 1-(mesitylene-2-sulfonyl)-3-nitro-1,2,4-triazole (MSNT) as condensing agent,

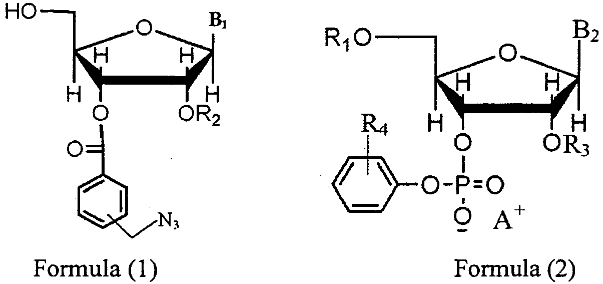

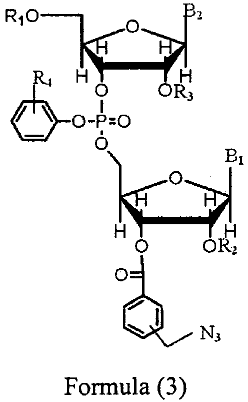

Wherein:
R₁ represents 4,4'-dimethoxytriphenylmethyl, RNA or DNA;
R₂ and R₃ independently represent a sterically hindered silane protective group;
R₄ represents a halogen atom;
A⁺ represents a tri-alkyl ammonium ion;
B₁ and B₂ independently represent guanyl substituted with N-acyl, adeninyl substituted with N-acyl, cytosine substituted with N-acyl, thymine or uracil.

This step may be represented by Reaction Scheme I:

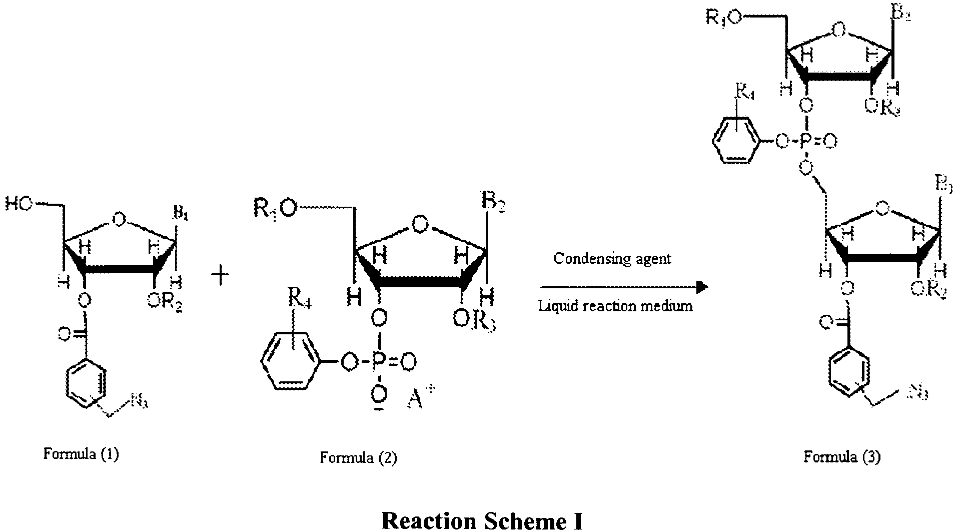

The condition of the condensation reaction includes the use of MSNT (1-(mesitylene-2-sulfonyl)-3-nitro-1,2,4-triazole) as condensing agent. MSNT is represented by Formula (8):

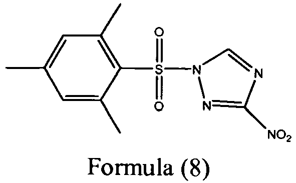

The reaction medium may be pyridine.

Relative to 1mol of the compound represented by Formula (1), the use amount of the compound represented by Formula (2) may be 0.8-3mol, preferably 1-2mol and more preferably 1-1.3mol; the use amount of the condensing agent may be 2-5mol and preferably 2.5-3mol; the use amount of the reaction medium may be 5-50L and preferably 5-20L.

The reaction temperature of the reaction shown in Reaction Scheme I may be 10-50°C and preferably 20-35 °C; the reaction time may be 0.5-10h and preferably 1-5h.

In Formulae (1), (2) and (3), the sterically hindered silane protective group may be any kind of silane groups with the functions of steric hindrance and protection. The preferred may be tert-butyl dimethyl silyl, phenyl dimethyl silyl, tert-butyl diphenyl silyl or triisopropyl silyl. The more preferred may be tert-butyl dimethyl silyl.

The halogen atom may be F, Cl, Br or I. The preferred may be Cl or Br. The more preferred may be Cl.

The alkyl groups in the tri-alkyl ammonium ion may be same or different and may each have 1-6 carbon atoms, and preferably 1-4 carbon atoms. The particularly preferred alkyl groups include but are not limited to methyl, ethyl, n-propyl, iso-propyl, n-butyl, iso-butyl and tert-butyl.

The acyl in B₁ and B₂ may be acyl containing 2-10 carbon atoms. The preferred may be benzoyl, isobutyryl or acetyl.

In Formula (1) or (3), -CH₂-N₃ may be at ortho-, meta- or para- position, and preferably at ortho-position.

In Formula (2) or (3), R₄ may be in ortho-, meta- or para- position, and preferably at ortho-position.

After the reaction in Reaction Scheme I is completed, the reaction may be terminated and the product is separated.

The process for terminating the reaction may include: mixing the reaction solution with an aqueous solution of triethylammonium bicarbonate (TEAB) and holding for 10-90min under stirring. The concentration of TEAB may be 0.1-1mol/L. The ratio by volume of TEAB to the reaction medium may be 0.02-0.5.

The separation method may include: mixing the reaction solution with dichloromethane after the reaction is terminated, adding TEAB to wash it, and subjecting the organic phase to drying, filtering, concentrating and separating in a normal-pressure column to obtain the product. The ratio by volume of dichloromethane to the reaction medium may be 2-20. The concentration of TEAB may be 0.05-0.5mol/L. The washing may be performed one or more times. The ratio between the total volume of TEAB for washing and the volume of the reaction medium may be 2-20. The drying, filtration, concentration and normal-pressure column separation methods are well-known to those skilled in the art and will not be detailedly described here.

If necessary, after the reaction in Reaction Scheme I is completed, the 5'-protective groups of the obtained DNA or RNA short chains may be removed (the second-step reaction shown in Reaction Scheme II), condensation reaction with the new compound represented by Formula (2) takes place again to obtain longer chains, the reaction is repeated several times and finally all protective groups are removed such that the oligonucleotides with the needed length are obtained; alternatively, the 3'-protective groups of the newly obtained DNA or RNA short chains may be removed, then 3'-hydroxyl phosphate is esterified to generate a new P-component (the reaction shown in Reaction Scheme III), it may again take condensation reaction with the new compound represented by Formula (3) to obtain longer chains, the reaction is repeated several times and finally all protective groups are removed such that the expected oligonucleotides are obtained.

According to the method provided by the present invention, the compound represented by Formula (1) may be obtained from the reaction shown in Reaction Scheme II. That is, the compound represented by Formula (4) reacts with the compound represented by Formula (5) under the condensation reaction condition and then 5'-R₅ protective group is removed.

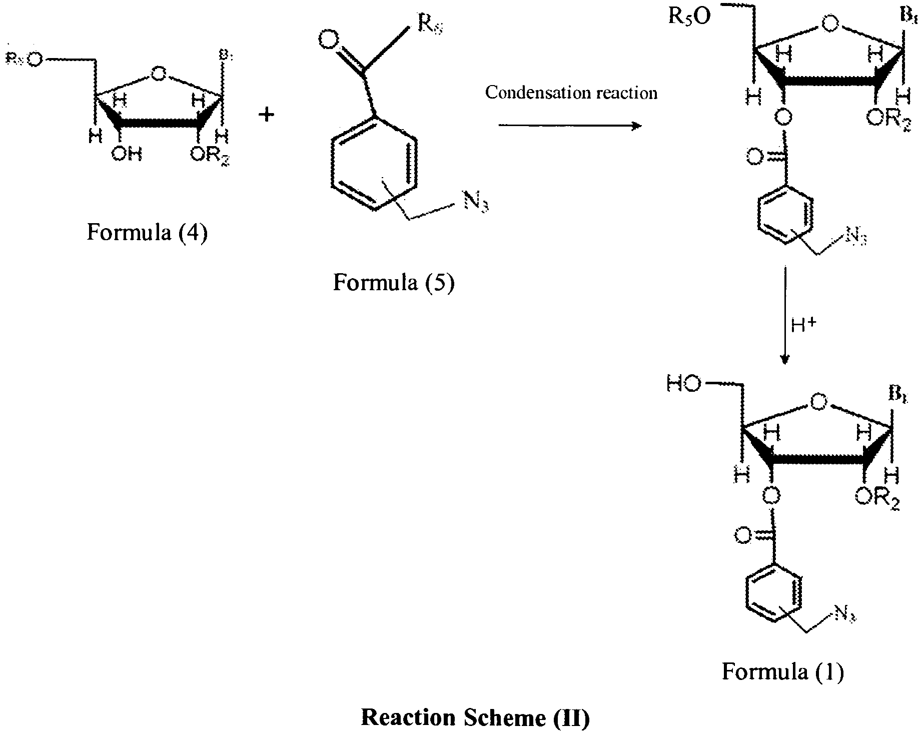

In Formula (4) and Formula (5), R₂ represents a sterically hindered silane protective group. It may be any kind of silane group with the functions of steric hindrance and protection. The preferred one may be tert-butyl dimethyl silyl, phenyl dimethyl silyl, tert-butyl diphenyl silyl or triisopropyl silyl. The more preferred one may be tert-butyl dimethyl silyl.

R₅ represents DMTr, RNA or DNA.

R₆ represents a halogen atom. The halogen atom may be F, Cl, Br or I. The preferred one may be Cl or Br. The more preferred one may be Cl.

B₁ represents guanyl substituted with N-acyl, adeninyl substituted with N-acyl, cytosine substituted with N-acyl, thymine or uracil. The acyl may be acyl containing 2-10 carbon atoms.

The preferred one may be benzoyl, isobutyryl or acetyl.

In Formula (1) or Formula (4), -CH₂-N₃ may be at ortho-, meta- or para- position, and preferably at ortho-position.

In Reaction Scheme II, the condensation reaction condition may include: using N-methylimidazole as an adjuvant; and using one or more of dichloromethane and pyridine as reaction medium.

Relative to 1mol of the compound represented by Formula (4), the use amount of the compound represented by Formula (5) may be 1-3mol and preferably 1.5-3mol; the use amount of the adjuvant may be 1-8mol and preferably 2-3.5mol; the use amount of the reaction medium may be 20-200L and preferably 30-150L.

The reaction temperature may be -10°C~10°C and the reaction is preferably performed in an ice bath; the reacion time may be 5-100h and preferably 10-60h.

After the condensation reaction in Reaction Scheme II is completed, the reaction may be terminated and the product of the condensation reaction is separated.

The reaction termination method may include: mixing the reaction solution with a saturated aqueous solution of NaHCO₃ for 1-20min. The ratio by volume of the saturated aqueous solution of NaHCO₃ to the reaction medium may be 1-10.

The separation method may include: separating the organic phase and water phase of the reaction solution after the reaction is terminated, washing the organic phase with a saturated aqueous solution of NaHCO₃ and subjecting the organic phase to drying, filtering, concentrating and separating in a normal-pressure column to obtain the product. The washing may be performed one or more times. The ratio between the total volume of the saturated aqueous solution of NaHCO₃ and the volume of the reaction medium may be 1-20. The drying, filtration, concentration and normal-pressure column separation methods are well-known to those skilled in the art and will be not detailedly described here.

The method to remove 5'-R₅ protective group may include: the product of the condensation reaction reacts with formic acid in chloroform under stirring at 10-50 °C for 5-60min. After the reaction, the organic phase and the formic acid phase may be directly separated, the formic acid phase is extracted with dichloromethane, and all organic phase is dried, filtered, concentrated, and separated in a normal-pressure column to obtain the product. The extraction may be performed one or more times. The ratio between the total volume of dichloromethane used during extraction and the volume of formic acid may be 1-20. The drying, filtration, concentration and normal-pressure column separation methods are well-known to those skilled in the art and will not be detailedly described here. Relative to 1 mol of the product of the condensation reaction, the use amount of formic acid may be 10-50L.

The method to remove 5'-R₅ protective group may also include: stirring the condensation product in 1% p-toluene sulfonic acid or 3%-5% trichloroacetic acid or 3%-5% trifluoroacetic acid in dichloromethane 1-60min. The number of equivalents of the contained organic acids is 5-20 times of the reaction stoichiometric number. After the reaction is completed, the solution is neutralized with a saturated aqueous solution of NaHCO₃ and the organic phase is separated. The product is obtained after the organic phase is dried, filtered, concentrated, and separated in a normal-pressure column. The drying, filtration, concentration and normal-pressure column separation methods are well-known to those skilled in the art and will not be detailedly described here.

The method to remove 3'-protective group may include: the condensation product reacts with triphenylphosphine or diphenyl methyl phosphine in dioxane containing 10% water under stirring at 10-50°C overnight. After the reaction is completed, the solvent will be evaporated. The product may be obtained through separation in a normal-pressure column. Relative to 1 mol of the product of the condensation reaction, the use amount of triphenylphosphine or diphenyl methyl phosphine may be 3-5mol and the use amount of dioxane may be 50-150L. The distillation and normal-pressure column separation methods are well-known to those skilled in the art and will not be detailedly described here.

The preparation method of the compound represented by Formula (5) may include the following steps:
(1) in the presence of benzoyl peroxide, the compound represented by Formula (9) reacts with N-halogenated succinimide to obtain the compound represented by Formula (10);
(2) the compound represented by Formula (10) reacts with alkali metal azide to obtain the compound represented by Formula (11);
(3) the compound represented by Formula (11) is hydrolyzed and acylated to obtain the compound represented by Formula (5),

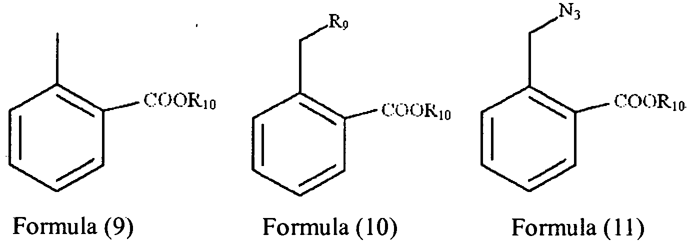

Where: R₉ represents a halogen atom; R₁₀ represents C₁-C₄ alkyl.

In step (1), the reaction medium may be one or more of carbon tetrachloride, chloroform, benzene, toluene and heptane. Relative to 1mol of the compound represented by Formula (9), the use amount ofN- halogenated succinimide may be 1-3mol and preferably 1-1.2mol; the use amount of benzoyl peroxide may be 0.01-0.1mol and preferably 0.1-0.2mol; the use amount of the reaction medium may be 5-20L and preferably 6-10L; the reaction temperature may be 80~120°C and preferably 90°C~110°C; and the reaction time may be 0.5-6h and preferably 1-3h. In step (2), the reaction medium may be one or more of ethanol, acetone, N, N-dimethyl formamide and dimethyl sulfoxide. Relative to 1mol of the compound represented by Formula (10), the use amount of alkali metal azide may be 1-3mol and preferably 1.5-2mol; the use amount of the reaction medium may be 3-10L and preferably 6-8L; the reaction temperature may be 0°C~80°C, and preferably 25°C~35°C; and reaction time may be 2-30h.

In step (3), the hydrolysis includes the reaction between the compound represented by Formula (11) and the alcohol-water mixed solution (volume ratio 1 : 1) of alkali metal hydroxide. Relative to 1mol of the compound represented by Formula (10), the use amount of alkali metal hydroxide may be 5-100mol and preferably 10-20mol; the reaction temperature may be 0°C~50°C and preferably 25°C~35°C; the reaction time may be 0.1-2h and preferably 0.25-1h; and the concentration of alkali metal hydroxide in the alcohol-water mixed solution may be 5-10wt%.

The compound represented by Formula (2) may be obtained from the reaction shown in Reaction Scheme III, i.e.: the reaction of the compound represented by Formula (6), the compound represented by Formula (7) and trialkyl amine in a reaction medium in the presence of a catalyst,

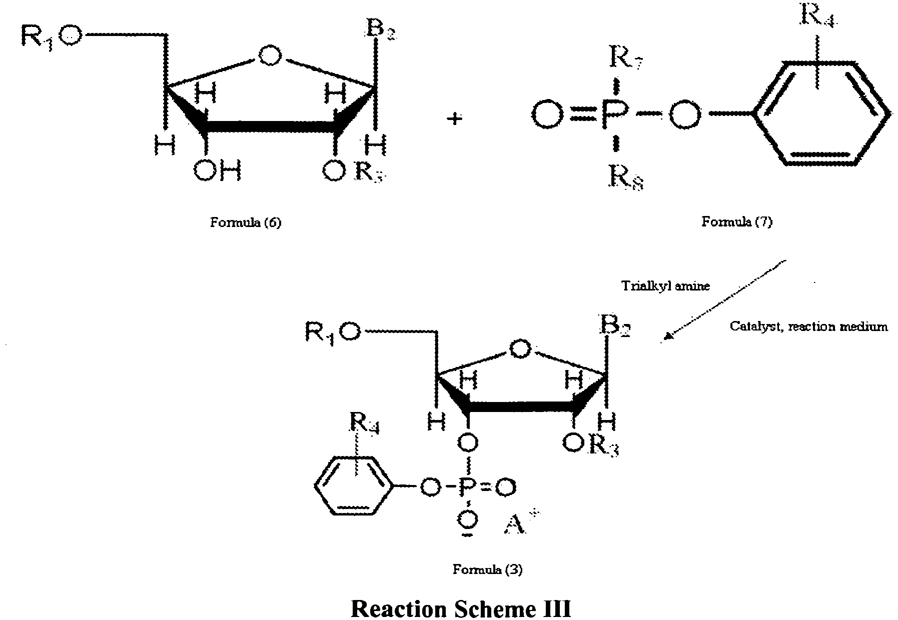

The catalyst may be one or more of 1,2,4-triazole, triethylamine and pyridine.

The reaction medium may be one or more of dichloromethane, dioxane and tetrahydrofuran.

In Formula (6) and Formula (7), R₁ represents DMTr, RNA or DNA;

R₃ represents a sterically hindered silane protective group and may be any kind of silane group with the functions of steric hindrance and protection, in which the preferred one may be tert-butyl dimethyl silyl, phenyl dimethyl silyl, tert-butyl diphenyl silyl or triisopropyl silyl and the more preferred one may be tert-butyl dimethyl silyl.

R₄, R₇ and R₈ each independently represent a alogen atom. The halogen atoms may be F, Cl, Br or I. The preferred may be Cl or Br. The more preferred may be Cl.

B₂ represents guanyl substituted with N-acyl, adeninyl substituted with N-acyl, cytosine substituted with N-acyl, thymine or uracil. The acyl may be an acyl containing 2-10 carbon atoms. The preferred one may be benzoyl, isobutyryl or acetyl.

In Formula (3) or Formula (7), R₄ may be at ortho-, meta- or para- position, and preferably at ortho- position.

The condition of the reaction shown in Reaction Scheme III may include: the reaction temperature may be -10□~10□ and the reaction is preferably performed in an ice bath; the reaction may be divided into two steps, the reaction time in the first step may be 0.5-10h and preferably 1-3h.

Relative to 1mol of the compound represented by Formula (6), the use amount of the compound represented by Formula (7) may be 1-5mol and preferably 1.5-3mol; the use amount of trialkyl amine may be 1-50mol and preferably 3-20mol; the use amount of catalyst may be 2-10mol and preferably 2-6mol; the use amount of the reaction medium may be 5-200L and preferably 10-100L.

After the reaction in the first step is completed, the reaction solution and TEAB are mixed and held for 10-90min under stirring. The concentration of TEAB may be 0.1-1mol/L and the ratio by volume of TEAB to the reaction medium may be 0.2-2; then the organic phase and water phase are directly separated, the organic phase is washed with TEAB and the product may be obtained through drying, filtering, concentrating and separating in a normal-pressure column the organic phase. The concentration of the TEAB for washing may be 0.1-1mol/L. The washing may be performed one or more times. The ratio by volume of the TEAB for washing to the reaction medium may be 0.5-5.

Hereinafter the present invention will be further described with reference to the examples.

The raw materials used in the examples are as follows:
Nucleotide 1 (A): The compound represented by Formula (6), protected adenine ribonucleotide, in which B₂ is N-benzoyl adenine, R₁ is DMTr and R₃ is tert-butyl dimethyl silyl. It is purchased from Shanghai GenePharma Co., Ltd.;
Nucleotide 2 (A): The compound represented by Formula (4), protected adenine ribonucleotide, in which B₁ is N-benzoyl adenine, R₅ is DMTr and R₂ is tert-butyl dimethyl silyl. It is purchased from Shanghai GenePharma Co., Ltd.;
Nucleotide 3 (C): The compound represented by Formula (6), protected cytosine ribonucleotide, in which B₂ is N-benzoyl cytosine, R₁ is DMTr and R₃ is tert-butyl dimethyl silyl. It is purchased from Shanghai GenePharma Co., Ltd.;
Nucleotide 4 (G): The compound represented by Formula (4), protected guanine ribonucleotide, in which B₁ is N-acetyl guanine, R₅ is DMTr and R₂ is tert-butyl dimethyl silyl. It is purchased from Shanghai GenePharma Co., Ltd.;
Nucleotide 5 (G): The compound represented by Formula (6), protected guanine ribonucleotide, in which B₂ is N-benzoyl guanine, R₁ is DMTr and R₃ is tert-butyl dimethyl silyl. It is purchased from Shanghai GenePharma Co., Ltd.;
Nucleotide 6 (U): The compound represented by Formula (4), protected uracil ribonucleotide, in which B₁ is uracil, R₅ is DMTr and R₂ is tert-butyl dimethyl silyl. It is purchased from Shanghai GenePharma Co., Ltd.;
Nucleotide 7 (U): The compound represented by Formula (6), protected uracil ribonucleotide, in which B₂ is uracil, R₁ is DMTr and R₃ is tert-butyl dimethyl silyl. It is purchased from Shanghai GenePharma Co., Ltd.;
Triethylamine: Purchased from Tianjin Beifang Tianyi Chemical Reagents Factory; 1,2,4-triazole: Purchased from Alfa Aesar;
2-chlorophenyl dichlorophosphate: The compound represented by Formula (7), in which R₄, R₇ and R₈ are all Cl and R₄ is at ortho-position. It is purchased from Alfa Aesar;
o-azido methyl benzoyl chloride: The compound represented by Formula (5), in which R₆ is Cl and at ortho-position. It is obtained in the synthesis example 6.
MSNT: The compound represented by Formula (8), purchased from Sigma Aldrich.

### Synthesis example 1

This synthesis example is intended to prepare the raw material of the compound represented by Formula (3), i.e.: the compound (P-component) represented by Formula (2).

Add 1,2,4-triazole (2.76g, 40mmol) and triethylamine (10.1g, 100mmol) into a 250ml round bottom flask, dissolve them in 15ml of dichloromethane, dropwise add 10ml of dichloromethane solution containing 2-chlorophenyl dichlorophosphate (4.91g, 20mmol) in ice bath, then dropwise add 35ml of dichloromethane solution containing nucleotide 1 (7.87g, 10mmol), stir for 2.5h in ice bath, then add 35ml of 1M TEAB, continue to stir for 0.5h, then add 1M TEAB to extract it three times (20ml each time), dry all organic phase over anhydrous sodium sulfate, filter it and remove the solvent through rotary evaporation to obtain 10.78g of the product with a yield of 100%. ³¹PNMR (CDCl₃, 121M)δ-6.09. ESI-MS, M- 976.2926. The yield is the percentage between the weight of the product and the calculated theoretical output of nucleotide 1.

### Synthesis example 2

This synthesis example is intended to prepare the raw material of the compound represented by Formula (3), i.e.: the compound (OH-component) represented by Formula (1).

Add N-methyl imidazole (2.08g, 25mmol) into a 250ml round bottom flask, dissolve it in 15ml of dichloromethane, dropwise add 15ml of dichloromethane solution containing o-azido methyl benzoyl chloride (4.18g, 20mmol) in ice bath, then dropwise add 40ml of dichloromethane solution containing nucleotide 2 (7.87g, 10mmol), continue to react in ice bath for 40h and add 100ml of saturated NaHCO₃ to terminate the reaction. After liquid separation, the organic phase is washed two times with saturated NaHCO₃ (100ml each time), dried over anhydrous sodium sulfate, filtered, concentrated and separated in a normal-pressure column to obtain 8.21g of the product with a yield of 86.8%. The yield is the percentage between the weight of the product and the calculated theoretical output of nucleotide 2.

Add and dissolve the product obtained above (8.21g, 8.67mmol) into 170ml of chloroform, add 170ml of HCOOH under quick stirring (stirring speed 500-1000rpm), react at room temperature for 30min, directly separate the solution, extract the HCOOH phase with dichloromethane three more times (170ml of dichloromethane each time), combine the extraction liquid, wash it with water three times (170ml of water each time), wash it with 0.1M TEAB one time (170ml), dry the organic phase over anhydrous sodium sulfate and then filter, concentrate and separate in a normal-presure column the organic phase to obtain 4.78g of the product with a yield of 85.5%. The yield is the percentage between the weight of the product and the calculated theoretical output of nucleotide 2.

HNMR(CDCl₃, 300M)δ(-0.32, 3H, 1CH₃), (-0.00, 3H, 1CH₃), (-0.77, 9H, 3CH₃), 1.89(s, 1H), 4.01-4.28(dd, 2H, CH₂), 4.99(s, 2H, CH₂), 5.42-5.46(t, 1H), 5.88, 5.90(d, 1H), 6.09, 6.12(d, 1H), 6.26(d, 1H), 7.37(s, 1H), 7.58-7.79(m, 5H), 8.16-8.31(m, 4H), 8.98(s, 1H), 9.22(s, 1H), ESI-MS: (M+Na)⁺667.2419

### Synthesis example 3

The synthesis is performed in the same manner as the synthesis example 1, except that nucleotide 1 is substituted with nucleotide 3.

### Synthesis example 4

The synthesis is performed in the same manner as the synthesis example 2, except that nucleotide 2 is substituted with nucleotide 4.

### Synthesis example 5

The synthesis is performed in the same manner as the synthesis example 1, except that nucleotide 1 is substituted with nucleotide 5.

### Synthesis example 6

The synthesis is performed in the same manner as the synthesis example 1, except that nucleotide 1 is substituted with nucleotide 7.

### Synthesis example 7

The synthesis is performed in the same manner as the synthesis example 2, except that nucleotide 2 is substituted with nucleotide 6.

### Synthesis example 8

The synthesis is performed in the same manner as the synthesis example 2, except that nucleotide 2 is substituted with nucleotide 3.

### Synthesis example 9

This synthesis example synthesizes the compound represented by Formula (5).
(1) Add 10.0g of compound 1 into a 250ml round bottom flask provided with a reflux device, add 80ml of SOCl₂, heat and reflux the solution and react under stirring at 90□ for 5h. Change the reflux device into a distiller and distill out solvent SOCl₂. Add 60ml of methanol after cooling, dropwise add 15ml of triethylamine at room temperature under stirring and continue the reaction at room temperature for 30min under stirring. Convert all acyl chloride into ester, remove excessive methanol and triethylamine through rotary evaporation, add 50ml of ethyl acetate, wash it with water twice and with saturated NaCl water solution once and then dry the ester layer over anhydrous Na₂SO₄. After the solvent is removed, 9.7g of compound 2 is obtained with a yield of 88.2%. 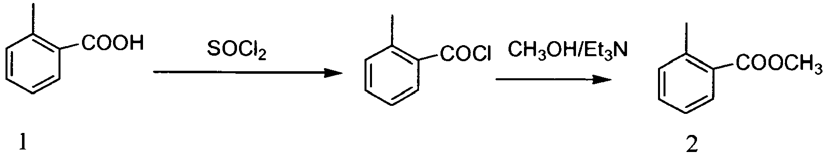
(2) Add 1.50g (10mmol) of compound 2 into a 250ml round bottom flask, dissolve it in 80ml of CCl₄, then add 1.98g (11mmol) of NBS and 48mg (0.2mmol) of BPO and heat and reflux for 1.5h to complete reaction. Remove the floating insolubles through filtration and then remove the solvent to obtain compound 3. The reaction is quantitative. 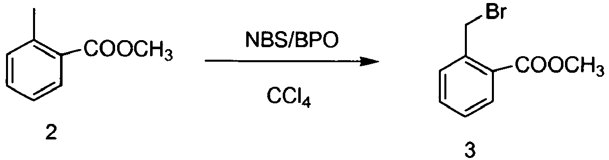 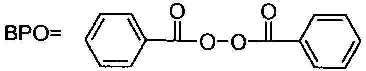 BPO= Benzoyl peroxide 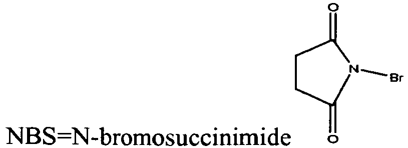
(3) Add 916mg (4mmol) of compound 3 into a 250ml round bottom flask, dissolve it in 20ml of ethanol, then add 520mg (8mmol) of NaN₃ and stir at room temperature overnight (21h). Remove ethanol through rotary evaporation, add ethyl acetate to dissolve it, wash it with water and saturated NaCl water solution in turn, remove solvent from the organic layer through rotary evaporation, add 40ml of 5% NaOH solution (CH₃OH:H₂O=1:1 v/v), stir at room temperature for 30min, remove methanol through rotary evaporation, regulate pH to about 1 with 2.5M HCl and extract with CH₂Cl₂ 4 times. Dry the organic phase over anhydrous Na₂SO₄ and remove solvent to obtain compound 5. Re-crystallize compound 5 with cyclohexane to obtain white crystal.
   NMR data of compound 5:
   ¹HNMR(CDCl₃,400M),4.89,(s,2H,CH₂),7.43-7.47(t,1H),7.54-7.56(d, 1H), 7.61-7.64(t, 1H), 8.18-8.20(d,1H).
   Element analysis: C: 53.80, H: 3.99, N: 23.92, calculated value: C: 54.24, H: 3, 98, N23.72 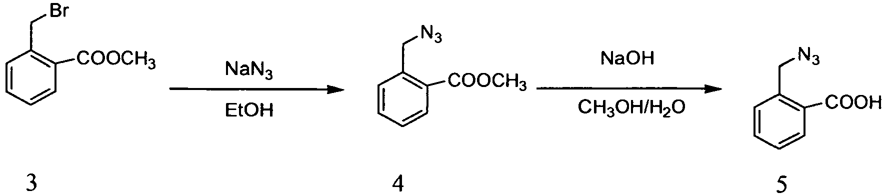
(4) Add 1.0g (5.65mmol) of compound 5 into a 100ml round bottom flask, dissolve it in 50ml of CHCl₃, then add 2.02g (16.95mmol) of SOCl₂ and heat and reflux for 5h. Remove the solvent through rotary evaporation after the reaction is stopped, bring out excessive SOCl₂ through azetropy of toluene 3×3ml to obtain compound 6, and store it in a desiccator for future use.

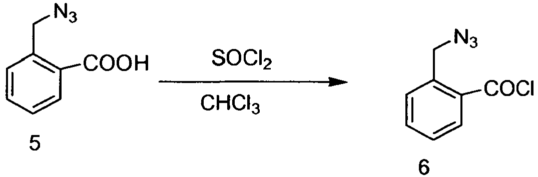

### Example 1

This example synthesizes oligonucleotides.
Synthesis target: Fully protected RNA
DMTr [CGAAAGAACG] AZMB
Note: [] indicates fully protected RNA except 3' and 5' ends
DMTr is 4,4'-dimethoxytriphenylmethyl, a protective group at 5' end;
AZMB is o-azido methyl benzoyl chloride, a protective group at 3' end.

### (1) Synthesis of DMTr [CpG] AZMB

Add the product of synthesis example 4 (598mg, 1mmol) and the product of synthesis example 3 (1.19g, 1.2mmol) into a 25ml round bottom flask, add 10ml of anhydrous pyridine to dissolve the sample, add MSNT by three times (745mg, 2.8mmol in total) and stir at room temperature for 3h. Add 1ml of 1M TEAB, stir for 0.5h to terminate the reaction, pour the reaction solution into 60ml of dichloromethane, wash it with 0.1M TEAB three times (20ml each time), dry the organic phase over anhydrous sulfate and filter, concentrate and separate in a normal-pressure column to obtain 831mg of the product with a yield of 56.4%. The yield is the percentage between the weight of the product and the calculated theoretical output calculated according to the product of synthesis example 4.

### (2) Synthesis of OH-[CpG]AZMB

Dissolve DMTr[CG]AZMB (800mg, 0.54mmol) obtained in step (1) in 7.5ml of chloroform, add 7.5ml of HCOOH under quick stirring, react at room temperature for 30min, directly separate the solution, extract the HCOOH phase with dichloromethane three times (5mL each time), combine the extraction liquid, wash it with water three times (5ml each time) and with 0.1M TEAB (10ml) once. Dry the organic phase over anhydrous sodium sulfate and then filter, concentrate and separate in a normal-presure column the organic phase to obtain 500mg of the product with a yield of 79.1%. The yield is the percentage between the weight of the product and the calculated theoretical output of DMTr[CG]AZMB.

### (3) Synthesis of DMTr[AA]AZMB

Add the product of synthesis example 2 (1.39g, 2.16mmol) and the product of synthesis example 1 (3.03g, 2.81mmol) into a 100ml round bottom flask, add 22ml of anhydrous pyridine to dissolve the sample, add MSNT (1.80g, 6.05mmol) by three times and stir at room temperature for 2h. Add 1M TEAB, stir for 0.5h to terminate the reaction, pour the reaction solution into 150ml of dichloromethane, wash it with 0.1M TEAB three times, dry the organic phase over anhydrous sodium sulfate and filter, concentrate and separate in a normal-pressure column to obtain 3.08g of the product with a yield of 87.9%. The yield is the percentage between the weight of the product and the calculated theoretical output calculated according to the product of synthesis example 2.

### (4) Synthesis of OH[AA]AZMB

Dissolve DMTr[AA]AZMB (3.08g, 1.92mmol) obtained in step (3) in 100ml of 1% p-toluene sulfonic acid solution, stir at room temperature for 30min, separate the organic phase after neutralization with saturated NaHCO₃, extract the water phase with dichloromethane twice, combine the organic phase, dry the organic phase over anhydrous sodium sulfate and then filter, concentrate and separate in a normal-presure column the organic phase to obtain 2.45g of the product with a yield of 98.8%. The yield is the percentage between the weight of the product and the calculated theoretical output calculated according to DMTr[AA]AZMB.

### (5) Synthesis of DMTr[GAA]AZMB

Add OH[AA]AZMB (2.45g, 1.88mmol) obtained in step (4) and the product of synthesis example 5 (2.39g, 2.26mmol) into a 100ml round bottom flask, add 19ml of anhydrous pyridine to dissolve the sample, add MSNT (1.56g, 5.26mmol) by three times and stir at room temperature for 2h. Add 2ml of 1M TEAB, stir for 0.5h to terminate the reaction, pour the reaction solution into 200ml of dichloromethane, wash it with 0.1M TEAB three times, dry the organic phase over anhydrous sodium sulfate and filter, concentrate and separate in a normal-pressure column the organic phase to obtain 4.068g of the product with a yield of 96.0%. The yield is the percentage between the weight of the product and the calculated theoretical output calculated according to OH[AA]AZMB.

### (6) Synthesis of OH[GAA]AZMB

Dissolve DMTr[GAA]AZMB (4.068g, 1.81mmol) obtained in step (5) in 50ml of chloroform, add 50ml of HCOOH under quick stirring, react at room temperature for 30min, directly separate the solution, extract the HCOOH phase with dichloromethane three times, combine the extraction liquid, wash it with water three times and with 0.1M TEAB once, dry the organic phase over anhydrous sodium sulfate and filter, concentrate and separate in a normal-pressure column to obtain 2.66g of the product with a yield of 75.6%. The yield is the percentage between the weight of the product and the calculated theoretical output calculated according to DMTr[GAA]AZMB.

### (7) Synthesis of DMTr[AGAA]AZMB

Add OH[GAA]AZMB (1.48g, 0.76mmol) obtained in step (6) and the product of synthesis example 1 (1.08g, 1.00mmol) into a 100ml round bottom flask, add 8ml of anhydrous pyridine to dissolve the sample, add MSNT (632mg, 2.13mmol) by three times and stir it at room temperature for 4h. Add 1.5ml of 1M TEAB, stir for 0.5h to terminate the reaction, pour the reaction solution into 60ml of dichloromethane, wash it with 0.1M TEAB three times, dry the organic phase over anhydrous sodium sulfate and filter, concentrate and separate in a normal-pressure column the organic phase to obtain 2.21 g of crude product.

### (8) Synthesis of DMTr[AGAA]OH

Add DMTr[AGAA]AZMB (2.21g, 0.76mmol) obtained in step (7) into a 100ml round bottom flask, add 2.6g mixed solvent of dioxane/water (9:1) to dissolve it, add triphenylphosphine (796mg, 3.04mmol), stir at room temperature for 24h, evaporate the solvent and separate in a normal-pressure column to obtain 2.1g of crude product.

### (9) Synthesis of DMTr[AGAA]PO⁻

Add 1,2,4-triazole (420mg, 6.08mmol) and triethylamine (768mg, 7.60mmol) into a 100ml round bottom flask, dissove them in 7ml of dichloromethane, dropwise add 3ml of dichloromethane solution containing 2-chlorophenyl dichlorophosphate (560mg, 2.28mmol) in ice bath, then dropwise add 2.5ml of dichloromethane solution containing DMTr[AGAA]OH (2.1g, 0.76mmol) obtained in step (8), stir in ice salt bath for 2.5h, add 3ml of TEAB to terminate the reaction, wash it with 0.1M TEAB three times, dry the organic phase over anhydrous sodium sulfate and filter, concentrate and separate in a normal-pressure column the organic phase to obtain 1.31g of the product with 56.7% of total yield in the three steps.

### (10) Synthesis of DMTr[CGAA]AZMB

Add OH[GAA]AZMB (1.12g, 0.58mmol) obtained in step (6) and the product of synthesis example 3 (746mg, 0.75mmol) into a 100ml round bottom flask, add 6ml of anhydrous pyridine to dissolve the sample, add MSNT (482mg, 1.62mmol) by three times and stir at room temperature for 4h. Add 1.5ml of 1M TEAB, stir for 0.5h to terminate the reaction, pour the reaction solution into 60ml of dichloromethane, wash it with 0.1M TEAB three times, dry the organic phase over anhydrous sodium sulfate and filter, concentrate and separate in a normal-pressure column the organic phase to obtain 1.63g of crude product.

### (11) Synthesis of DMTr[CGAA]OH

Add DMTr[CGAA]AZMB (1.60g, 0.57mmol) obtained in step (10) into a 100ml round bottom flask, dissolve it in 1.6g mixed solvent of dioxane/water (9:1), add triphenylphosphine (595mg, 2.27mmol), stir at room temperature for 24h, evaporate the solvent and separate in a normal-pressure column to obtain 1.5g of crude product.

### (12) Synthesis of DMTr[CGAA]PO⁻

Add 1,2,4-triazole (315mg, 4.56mmol) and triethylamine (576mg, 5.70mmol) into a 25ml round bottom flask, dissolve them in 5ml of dichloromethane, dropwise add 2ml of dichloromethane solution containing 2-chlorophenyl dichlorophosphate (420mg, 1.71mmol) in ice bath, then dropwise add 2ml of dichloromethane solution containing DMTr[CGAA]OH (1.5g, 0.57mmol) obtained in step (11), stir in ice salt bath for 2.5h, add 1.5ml of TEAB to terminate the reaction, wash it with 0.1M TEAB three times, dry the organic phase over anhydrous sodium sulfate and filter, concentrate and separate in a normal-pressure column the organic phase to obtain 1.12g of the product with 61.9% of total yield in the three steps.

### (13) Synthesis of DMTr[AGAACG]AZMB

Add OH[CG]AZMB (480mg, 0.41mmol) obtained in step (2) and DMTr[AGAA]PO⁻ (1.20g, 0.40mmol) obtained in step (9) into a 25ml round bottom flask, add 8ml of anhydrous pyridine to dissolve the sample, add MSNT (333mg, 1.12mmol) by three times and stir it at room temperature for 2.5h. Add 1ml of 1M TEAB, stir for 0.5h to terminate the reaction, pour the reaction solution into 60ml of dichloromethane, wash it with 0.1M TEAB three times, dry the organic phase over anhydrous sodium sulfate and filter, concentrate and separate in a normal-pressure column the organic phase to obtain 1.21g of the product with a yield of 73.4%.

### (14) Synthesis of OH[AGAACG]AZMB

Add DMTr[AGAACG]AZMB (1.21g, 0.29mmol) obtained in step (13), add 10ml of chloroform to dissolve it, add 10ml of HCOOH under quick stirring, react at room temperature for 30min, directly separate the solution, extract the HCOOH phase with dichloromethane three times, combine the extraction liquid, wash it with water three times and with 0.1 M TEAB one time, dry the organic phase over anhydrous sodium sulfate and filter, concentrate and separate in a normal-pressure column the organic phase to obtain 863mg of the product with a yield of 78.6%.

### (15) Synthesis of DMTr[CGAAAGAACG]AZMB

Add OH[AGAACG]AZMB (840mg, 0.22mmol) obtained in step (14) and DMTr[CGAA]PO⁻ (786g, 0.26mmol) obtained in step (12) into a 25ml round bottom flask, add 5ml of anhydrous pyridine to dissolve the sample, add MSNT (184mg, 0.62mmol) by three times and stir at room temperature for 4h. Add 1ml of 1M TEAB, stir for 0.5h to terminate the reaction, pour the reaction solution into 60ml of dichloromethane, wash it with 0.1M TEAB three times, dry the organic phase over anhydrous sodium sulfate and filter, concentrate and separate in a normal-pressure column the organic phase to obtain 1.20g of the product with a yield of 82.4%. MAIDI-TOF MS: 6560.787.

### Example 2

This example synthesizes oligonucleotides.

Synthesis target: 5'-CGAAAGAACG-3'
(1) Dissolve 46mg (0.28mmol) of 4-nitrobenzaldoxime and 32mg (0.28mmol) of tetra methyl guanidine in 0.6ml mixed solution of dioxane/water (v/v 1: 1), mix them well, add 13mg (2umol) of DMTr[CGAAAGAACG]AZMB obtained in example 1 and react at room temperature for 30h. This step is to remove the protective group on phosphine.
(2) Centrifuge and dry at 40°C under vacuum the reaction solution obtained in step (1), add 5ml of concentrated ammonia (25%-28%), heat it to 50□ and react 12h. This step is to remove protective group on base and 3'-end.
(3) Centrifuge and dry at 40°C under vacuum the reaction solution obtained in step (2), add 1ml of 80% acetic acid, react at room temperature 30min, centrifuge and dry again, dissolve the residue in 2ml of water, wash it with 2ml of ethyl acetate, and freeze and dry it at -80□ under vacuum into dry powder. This step is to remove 5'-protective group.
(4) Add 1ml of 1M TBAF tetrahydrofuran solution into the freeze-dry powder and react at 370 overnight. This step is to remove 2'-protective group. Add an equal volume of water into the fully reacted reaction solution to quench it, centrifuge and dry it at 40□ under vaccum to remove tetrahydrofuran, desalt the residue in sephadex G25 column, and collect the product that has ultraviolet absorption at 260nm to obtain aqueous solution of the deprotected oligo-RNA 5'-CGAAAGAACG-3'. Freeze dry it at -80□ under vacuum into white dry powdery solid. MAIDI-TOF MS: 3233.67.

### Example 3

This example synthesizes oligonucleotides.
Synthesis target: Fully protected RNA
DMTr[UGCGCGUUGAUGUGAGGUUCCUU]AZMB
And DMTr[GGAACCUCACAUCAACGCGCAUU]AZMB

### (1) Synthesis of OH[CG]AZMB

Add the product of synthesis example 3 (4.433g, 4.2mmol) and the product of synthesis example 4 (2.194g, 3.5mmol) into a 50ml round bottom flask, add 30ml of anhydrous pyridine to dissolve the sample, add MSNT (2.911g, 9.8mmol) and stir at room temperature for 2h. Add 4ml of 0.1 M TEAB, stir for 10min to terminate the reaction, remove the solvent through rotary evaporation, dissove the obtained product in 50ml of CH₂Cl₂, wash it with 0.1M TEAB 3 times (20ml each time), regulate pH value to 3 with 5% oxalic acid, separate the solution to obtain organic phase, dry the organic phase over anhydrous Na₂SO₄ and filter it to obtain 50ml of filtrate. Transfer the obtained filtrate into a 250ml round bottom flask, slowly add 50ml of 6% CF₃COOH, stir and react at room temperature for 5min, add saturated NaHCO₃ to neutralize the solution, separate the solution to obtain organic phase, wash the obtained organic phase with 20ml of saturated NaHCO₃ once, dry the organic phase over anhydrous sodium sulfate and filter, concentrate and separate in a normal-pressure column the organic phase to obtain the product with a yield of 78%. The yield is the percentage between the weight of the product and the calculated theoretical output calculated according to the product of synthesis example 4.

### (2) Synthesis of OH[GG]AZMB

The step same as step (1) is adopted to synthesize OH[GG]AZMB, except that the product of synthesis example 5 (4.471g, 4.2mmol) and the product of synthesis example 4 (2.194g, 3.5mmol) are adopted and dissolved in 20ml of anhydrous pyridine and the use amount of 0.1M TEAB is 3ml. The yield is 67.1 %. The yield is the percentage between the weight of the product and the calculated theoretical output calculated according to the product of synthesis example 4.

### (3) Synthesis of OH[AU]AZMB

Add the product of synthesis example 1 (4.534g, 4.2mmol) and the product of synthesis example 7 (1.811g, 3.5mmol) into a 50ml round bottom flask, add 20ml of pyridine to dissolve the sample, add MSNT (2.911g, 9.8mmol) by batch and stir and react at room temperature for 2h. Add 10ml of 1M TEAB, stir for 10min to terminate the reaction, dissove it in 100ml of CH₂Cl₂, wash it with 1M TEAB 3 times (20ml each time), separate the solution to obtain organic phase, dry the organic phase over anhydrous Na₂SO₄ and filter, concentrate and separate in a normal-pressure column the organic phase to obtain the product. Dissolve the obtained product in 45ml of CH₂Cl₂, slowly add 45ml of 6% CF₃COOH under stirring, ensure the concentration of CF₃COOH in the system is 3%, and stir and react at room temperature for 5min. Add saturated NaHCO₃ to neutralize the solution, separate the solution to obtain organic phase, wash the organic phase with 20ml of saturated NaHCO₃ once, dry the organic phase over anhydrous sodium sulfate and filter, concentrate and separate in a normal-pressure column the organic phase to obtain the product with a yield of 81.1%. The yield is the percentage between the weight of the product and the calculated theoretical output calculated according to the product of synthesis example 7.

### (4) Synthesis of OH[UU]AZMB

Add the product of synthesis example 6 (10.0g, 10.5mmol) and the product of synthesis example 7 (4.53g, 8.75mmol) into a 250ml round bottom flask, add 45ml of anhydrous pyridine to dissolve the sample, add MSNT (7.28g, 24.5mmol) by batch and stir and react at room temperature for 2h. Add 50ml of 1M TEAB, stir for 20min to terminate the reaction, dissolve it in 300ml of CH₂Cl₂, wash it with 1M TEAB 3 times (20ml each time), separate the solution to obtain an organic phase, dry the organic phase over anhydrous Na₂SO₄ and filter, concentrate and separate in a normal-pressure column the organic phase to obtain the product. Dissolve the obtained product in 100ml of CH₂Cl₂, slowly add 100ml of 6% CF₃COOH under stirring and stir and react at room temperature for 5min. Add saturated NaHCO₃ to neutralize the solution, separate the solution to obtain an organic phase, wash the organic phase with 50ml of saturated NaHCO₃ once, dry the organic phase over anhydrous sodium sulfate and filter, concentrate and separate in a normal-pressure column the organic phase to obtain the product with a yield of 73%. The yield is the percentage between the weight of the product and the calculated theoretical output calculated according to the product of synthesis example 7.

### (5) Synthesis of OH[CA]AZMB

Add the product of synthesis example 3 (31.66g, 30mmol) and the product of synthesis example 2 (16.12g, 25mmol) into a 500ml round bottom flask, use 150ml of anhydrous pyridine to dissolve the sample, add MSNT (20.79g, 70mmol) by batch and stir and react at room temperature 2h. Add 10ml of 1M TEAB, stir 20min to terminate the reaction, dissolve it in 600ml of CH₂Cl₂, wash it with 1M TEAB 3 times (20ml each time), separate the solution to obtain an organic phase, dry the organic phase over anhydrous Na₂SO₄ and filter, concentrate and separate in a normal-pressure column the organic phase to obtain the product. Dissolve the obtained product in 370ml of CH₂Cl₂, slowly add 370ml of 6% CF₃COOH under stirring, ensure the concentration of CF₃COOH in the system is 3%, and stir and react at room temperature for 5min. Add saturated NaHCO₃ to neutralize the solution, separate the solution to obtain an organic phase, wash the organic phase with 20ml of saturated NaHCO₃ once, dry the organic phase over anhydrous sodium sulfate and filter, concentrate and separate in a normal-pressure column the organic phase to obtain the product with a yield of 72%. The yield is the percentage between the weight of the product and the calculated theoretical output calculated according to the product of synthesis example 2.

### (6) Synthesis of OH[CC]AZMB

A method same as step (4) is adopted to prepare OH[CC]AZMB, except that the product of synthesis example 3 (12.25g, 11.6mmol) and the product of synthesis example 8 (6.0g, 9.67mmol) are adopted, 50ml of anhydrous pyridine is used to dissolve the sample and MSNT (8.01g, 27.1mmol) is added by batch. The yield is 83%. The yield is the percentage between the weight of the product and the calculated theoretical output calculated according to the product of synthesis example 8.

### (7) Synthesis of OH[GC]AZMB

Add the product of synthesis example 5 (18.47g, 17.4mmol) and the product of synthesis example 8 (9.0g, 14.5mmol) into a 250ml round bottom flask, use 72ml of anhydrous pyridine to dissolve the sample, add MSNT (12.0g, 40.6mmol) by batch and stir and react at room temperature 2h. Add 80ml of 1M TEAB, stir 20min to terminate the reaction, dissolve it in 300ml of CH₂Cl₂, wash it with 1M TEAB 3 times (20ml each time), separate the solution to obtain an organic phase, dry the organic phase over anhydrous Na₂SO₄ and filter, concentrate and separate in a normal-pressure column the organic phase to obtain the product. Dissolve the obtained product in 150ml of CH₂Cl₂, slowly add 150ml of 6% CF₃COOH under stirring, ensure the concentration of CF₃COOH in the system is 3%, and stir and react at room temperature for 10min. Add saturated NaHCO₃ to neutralize the solution, separate the solution to obtain an organic phase, wash the organic phase with 120ml of saturated NaHCO₃ once and dry the organic phase over anhydrous Na₂SO₄. The yield is 63.8%. The yield is the percentage between the weight of the product and the calculated theoretical output calculated according to the product of synthesis example 8.

### (8) Synthesis of OH[UG]AZMB

Add the product of synthesis example 6 (5.14g, 5.4mmol) and the product of synthesis example 4 (2.82g, 4.5mmol) into a 50ml round bottom flask, use 30ml of anhydrous pyridine to dissolve the sample, add MSNT (3.74g, 12.6mmol) by batch and stir and react at room temperature for 2h. Add 10ml of 1M TEAB, stir for 20min to terminate the reaction, dissolve it in 150ml of CH₂Cl₂, wash it with 1M TEAB 3 times (20ml each time), separate the solution to obtain an organic phase, dry the organic phase over anhydrous Na₂SO₄ and filter, concentrate and separate in a normal-pressure column the organic phase to obtain the product. Dissolve the obtained product in 450ml of CH₂Cl₂, slowly add 450ml of 6% CF₃COOH under stirring, ensure the concentration of CF₃COOH in the system is 3%, and stir at room temperature 5min. Add saturated NaHCO₃ to neutralize the solution, separate the solution to obtain an organic phase, wash the organic phase with 300ml of saturated NaHCO₃ once, dry the organic phase over anhydrous sodium sulfate and filter, concentrate and separate in a normal-pressure column the organic phase to obtain the product with a yield of 78%. The yield is the percentage between the weight of the product and the calculated theoretical output calculated according to the product of synthesis example 4.

### (9) Synthesis of DMTr[GG]AZMB

Add the product of synthesis example 5 (7.64g, 7.2mmol) and the product of synthesis example 4 (3.76g, 6mmol) into a 100ml round bottom flask, add 35ml of anhydrous pyridine to dissolve the sample, add MSNT (5.0g, 16.8mmol) by three times and stir at room temperature 2.5h. Add 10ml of 1M TEAB, stir 20min to terminate the reaction, pour the reaction solution into 200ml of CH₂Cl₂, wash it with 1M TEAB three times (20ml each time), separate the solution to obtain an organic phase, dry the organic phase with Na₂SO₄ and filter, concentrate and separate in a normal-pressure column the organic phase to obtain the product with a yield of 81%. The yield is the percentage between the weight of the product and the calculated theoretical output calculated according to the product of synthesis example 4.

### (10) Synthesis of DMTr[UGC]AZMB

Add the product of synthesis example 6 (7.67g, 9.10mmol) and OH[GC]AZMB (7.77g, 6.17mmol) obtained in step (7) into a 250ml round bottom flask, add 40ml of anhydrous pyridine to dissolve the sample, add MSNT (5.13g, 17.3mmol) by three times and stir it at room temperature for 3.5h. Add 10ml of 1M TEAB, stir for 20min to terminate the reaction, pour the reaction solution into 150ml of CH₂Cl₂, wash it with 1M TEAB three times (20ml each time), dry the organic phase over Na₂SO₄ and filter, concentrate and separate in a normal-pressure column the organic phase to obtain the product with a yield of 73%. The yield is the percentage between the weight of the product and the calculated theoretical output calculated according to OH[GC]AZMB.

### (11) Synthesis of DMTr[UCA]AZMB

Add the product of synthesis example 6 (8.67g, 9.10mmol) and OH[CA]AZMB (8.95g, 7.00mmol) obtained in step (5) into a 50ml round bottom flask, add 50ml of anhydrous pyridine to dissolve the sample, add MSNT (5.82g, 19.6mmol) by three times and stir at room temperature 4h. Add 10ml of 1M TEAB, stir 20min to terminate the reaction, pour the reaction solution into 100ml of CH₂Cl₂, wash it with 1M TEAB three times (20ml each time), separate the solution to obtain an organic phase, dry the organic phase over Na₂SO₄ and filter, concentrate and separate in a normal-pressure column the organic phase to obtain the product with a yield of 77%. The yield is the percentage between the weight of the product and the calculated theoretical output calculated according to OH[CA]AZMB.

### (12) Synthesis of DMTr[ACG]AZMB

A method same as step (11) is adopted to prepare DMTr[ACG]AZMB, except that the product of synthesis example 1 (2.67g, 2.47mmol) and OH[CG]AZMB (2.40g, 1.90mmol) obtained in step (1) are adopted, 15ml of anhydrous pyridine is used to dissolve the sample and MSNT (1.58g, 5.32mmol) is added by three times. The yield is 78%. The yield is the percentage between the weight of the product and the calculated theoretical output calculated according to OH[CG]AZMB.

### (13) Synthesis of DMTr[GUU]AZMB

Add the product of synthesis example 5 (4.30g, 4.05mmol) and OH[UU]AZMB (3.15g, 3.00mmol) obtained in step (4) into a 100ml round bottom flask, use 20ml of anhydrous pyridine to dissolve the sample, add MSNT (2.50g, 8.40mmol) by three times and stir at room temperature 2.5h. Add 10ml of 1M TEAB, stir 20min to terminate the reaction, pour the reaction solution into 100ml of CH₂Cl₂, wash it with 1M TEAB three times (30ml each time), separate the solution to obtain an organic phase, dry the organic phase over Na₂SO₄ and filter, concentrate and separate in a normal-pressure column the organic phase to obtain the product with a yield of 79%. The yield is the percentage between the weight of the product and the calculated theoretical output calculated according to OH[UU]AZMB.

### (14) Synthesis of DMTr[GUG]AZMB

Add the product of synthesis example 5 (4.29g, 4.04mmol) and OH[UG]AZMB (3.60g, 3.11mmol) obtained in step (8) into a 100ml round bottom flask, use 20ml of anhydrous pyridine to dissolve the sample, add MSNT (2.59g, 8.71mmol) by three times and stir it at room temperature 3.5h. Add 10ml of 1M TEAB, stir 20min to terminate the reaction, pour the reaction solution into 100ml of CH₂Cl₂, wash it with 1M TEAB three times (20ml each time), separate the solution to obtain an organic phase, dry the organic phase over Na₂SO₄ and filter, concentrate and separate in a normal-pressure column the organic phase to obtain the product with a yield of 67% after purification by column chromatography. The yield is the percentage between the weight of the product and the calculated theoretical output calculated according to OH[UG]AZMB.

### (15) Synthesis of OH[ACC]AZMB

Add the product of synthesis example 1 (5.62g, 5.20mmol) and OH[CC]AZMB (5.4g, 4.33mmol) obtained in step (6) into a 100ml round bottom flask, use 22ml of anhydrous pyridine to dissolve the sample, add MSNT (3.59g, 12.1mmol) by batch and stir and react at room temperature 3h. Add 10ml of 1M TEAB, stir 20min to terminate the reaction, dissolve it in 150ml of CH₂Cl₂, wash it with 1M TEAB 3 times (20ml each time), separate the solution to obtain an organic phase, dry the organic phase over anhydrous Na₂SO₄ and filter, concentrate and separate in a normal-pressure column the organic phase to obtain the product. Dissolve the obtained product in 100ml of CH₂Cl₂, slowly add 100ml of 6% CF₃COOH under stirring, ensure the concentration of CF₃COOH in the system is 3%, and stir at room temperature for 30min. Add saturated NaHCO₃ to neutralize the solution, separate the solution to obtain an organic phase, wash the organic phase with 80ml of saturated NaHCO₃ once, dry the organic phase over anhydrous Na₂SO₄ and filter, concentrate and separate in a normal-pressure column the organic phase to obtain the product. The yield is 52%. The yield is the percentage between the weight of the product and the calculated theoretical output calculated according to OH[CC]AZMB.

### (16) Synthesis of OH[GCA]AZMB

Add the product of synthesis example 5 (4.00g, 3.77mmol) and OH[CA]AZMB (4.2g, 3.28mmol) obtained in step (5) into a 100ml round bottom flask, use 20ml of anhydrous pyridine to dissolve the sample, add MSNT (2.73g, 9.18mmol) by batch and stir and react at room temperature for 3h. Add 10ml of 1M TEAB, stir 20min to terminate the reaction, dissolve it in 150ml of CH₂Cl₂, wash it with 1M TEAB 3 times (20ml each time), separate the solution to obtain an organic phase, dry the organic phase over anhydrous Na₂SO₄ and filter, concentrate and separate in a normal-pressure column the organic phase to obtain the product. Dissolve the obtained product in 35ml of CH₂Cl₂, slowly add 35ml of 6% CF₃COOH under stirring, ensure the concentration of CF₃COOH in the system is 3%, and stir at room temperature 30min. Add saturated NaHCO₃ to neutralize the solution, separate the solution to obtain an organic phase, wash the organic phase with 25ml of saturated NaHCO₃ once, dry the organic phase over anhydrous Na₂SO₄ and filter, concentrate and separate in a normal-pressure column the organic phase to obtain the product with a yield of 68%. The yield is the percentage between the weight of the product and the calculated theoretical output calculated according to OH[CA]AZMB.

### (17) Synthesis of OH[UCC]AZMB

Add the product of synthesis example 6 (3.59g, 3.77mmol) and OH[CC]AZMB (2.42g, 2.90mmol) obtained in step (6) into a 50ml round bottom flask, add 20ml of anhydrous pyridine to dissolve the sample, add MSNT (2.41g, 8.12mmol) by batch and stir and react at room temperature for 5h. Add 10ml of 1M TEAB, stir for 20min to terminate the reaction, dissolve it in 100ml of CH₂Cl₂, wash it with 1M TEAB 3 times (20ml each time), separate the solution to obtain an organic phase, dry the organic phase over anhydrous Na₂SO₄ and filter, concentrate and separate in a normal-pressure column the organic phase to obtain the product. Dissolve the obtained product in 40ml of CH₂Cl₂, slowly add 40ml of 6% CF₃COOH under stirring, ensure the concentration of CF₃COOH in the system is 3%, and stir and react at room temperature for 30min. Add saturated NaHCO₃ to neutralize the solution, separate the solution to obtain an organic phase, wash the organic phase with 30ml of saturated NaHCO₃ once, dry the organic phase over anhydrous Na₂SO₄ and filter, concentrate and separate in a normal-pressure column the organic phase to obtain the product with a yield of 64.1%. The yield is the percentage between the weight of the product and the calculated theoretical output calculated according to OH[CC]AZMB.

### (18) Synthesis of OH[ACG]AZMB

Dissolve product DMTr[ACG]AZMB obtained in step (12) in 20ml of CH₂Cl₂, slowly add 20ml of 6% CF₃COOH under stirring, ensure the concentration of CF₃COOH in the system is 3%, and stir and react at room temperature for 10min. Add saturated NaHCO₃ to neutralize the solution, separate the solution to obtain an organic phase, wash the organic phase with 15ml of saturated NaHCO₃ once, dry the organic phase over anhydrous Na₂SO₄ and filter, concentrate and separate in a normal-pressure column the organic phase to obtain 2.0g of the product with a yield of 55.%. The yield is the percentage between the weight of the product and the calculated theoretical output calculated according to HO[CG]AZMB.

### (19) Synthesis of OH[GAU]AZMB

Add the product of synthesis example 5 (3.82g, 3.69mmol) and OH[AU]AZMB (3.30g, 2.80mmol) obtained in step (3) into a 100ml round bottom flask, add 20ml of anhydrous pyridine to dissolve the sample, add MSNT (2.33g, 7.84mmol) by three times and stir it at room temperature 4h. Add 5ml of 1M TEAB, stir 30min to terminate the reaction, pour the reaction solution into 100ml of CH₂Cl₂, wash it with 1M TEAB three times (20ml each time), separate the solution to obtain an organic phase, dry the organic phase over Na₂SO₄ and filter, concentrate and separate in a normal-pressure column the organic phase to obtain the product. Dissolve the obtained product in 40ml of CH₂Cl₂, slowly add 40ml of 6% CF₃COOH under stirring, ensure the concentration of CF₃COOH in the system is 3%, and stir and react at room temperature 30min. Add saturated NaHCO₃ to neutralize the solution, separate the solution to obtain an organic phase, wash the organic phase with 30ml of saturated NaHCO₃ once, dry the organic phase over anhydrous Na₂SO₄ and filter, concentrate and separate in a normal-pressure column the organic phase to obtain the product with a yield of 63%. The yield is the percentage between the weight of the product and the calculated theoretical output calculated according to OH[AU]AZMB.

### (20) Synthesis of OH[AGG]AZMB

Add the product of synthesis example 1 (3.30g, 3.10mmol) and OH[GG]AZMB (2.98g, 2.35mmol) obtained in step (2) into a 100ml round bottom flask, add 15ml of anhydrous pyridine to dissolve the sample, add MSNT (1.96g, 6.6mmol) by batch and stir and react at room temperature 2.5h. Add 10ml of 1M TEAB, stir 20min to terminate the reaction, pour the reaction solution into 150ml of CH₂Cl₂, wash it with 1M TEAB three times (20ml each time), separate the solution to obtain an organic phase, dry the organic phase over anhydrous Na₂SO₄ and filter, concentrate and separate in a normal-pressure column the organic phase to obtain the product. Dissolve the obtained product in 25ml of CH₂Cl₂, slowly add 25ml of 6% CF₃COOH under stirring, ensure the concentration of CF₃COOH in the system is 4%, and stir and react at room temperature 30min. Add saturated NaHCO₃ to neutralize the solution, separate the solution to obtain an organic phase, wash the organic phase with 20ml of saturated NaHCO₃ once, dry the organic phase over anhydrous Na₂SO₄ and filter, concentrate and separate in a normal-pressure column the organic phase to obtain the product with a yield of 69%. The yield is the percentage between the weight of the product and the calculated theoretical output calculated according to OH[GG]AZMB.

### (21) Synthesis of OH[AACC]AZMB

Add the product of synthesis example 1 (3.24g, 3.00mmol) and OH[ACC]AZMB (4.31g, 2.25mmol) obtained in step (15) into a 100ml round bottom flask, add 15ml of anhydrous pyridine to dissolve the sample, add MSNT (1.87g, 6.30mmol) by batch and stir and react at room temperature for 3h. Add 10ml of 1M TEAB, stir for 20min to terminate the reaction, pour the reaction solution into 100ml of CH₂Cl₂, wash it with 1M TEAB three times (20ml each time), separate the solution to obtain an organic phase, dry the organic phase over anhydrous Na₂SO₄ and filter, concentrate and separate in a normal-pressure column the organic phase to obtain the product. Dissolve the obtained product in 50ml of CH₂Cl₂, slowly add 50ml of 6% CF₃COOH under stirring, ensure the concentration of CF₃COOH in the system is 3%, and stir at room temperature 30min. Add saturated NaHCO₃ to neutralize the solution, separate the solution to obtain an organic phase, wash the organic phase with 40ml of saturated NaHCO₃ once, dry the organic phase over anhydrous Na₂SO₄ and filter, concentrate and separate in a normal-pressure column the organic phase to obtain the product with a yield of 50%. The yield is the percentage between the weight of the product and the calculated theoretical output calculated according to OH[ACC]AZMB.

### (22) Synthesis of DMTr[CGCA]AZMB

Add the product of synthesis example 3 (3.04g, 2.88mmol) and OH[GCA]AZMB (4.25g, 2.21mmol) obtained in step (16) into a 100ml round bottom flask, add 15ml of anhydrous pyridine to dissolve the sample, add MSNT (1.84g, 6.19mmol) by three times and stir it at room temperature 3.5h. Add 10ml of 1M TEAB, stir 20min to terminate the reaction, pour the reaction solution into 100ml of CH₂Cl₂, wash it with 1M TEAB three times (50ml each time), separate the solution to obtain an organic phase, dry the organic phase over anhydrous Na₂SO₄ and filter, concentrate and separate in a normal-pressure column the organic phase to obtain the product with a yield of 76%. The yield is the percentage between the weight of the product and the calculated theoretical output calculated according to OH[GCA]AZMB.

### (23) Synthesis of DMTr[UUCC]AZMB

Add the product of synthesis example 6 (2.43g, 2.55mmol) and OH[UCC]AZMB (3.50g, 1.96mmol) obtained in step (17) into a 50ml round bottom flask, add 15ml of anhydrous pyridine to dissolve the sample, add MSNT (1.63g, 5.49ml) by three times and stir it at room temperature for 4h. Add 10ml of 1M TEAB, stir for 20min to terminate the reaction, pour the reaction solution into 100ml of CH₂Cl₂, wash it with 1M TEAB three times (20ml each time), separate the solution to obtain an organic phase, dry the organic phase over anhydrous Na₂SO₄ and filter, concentrate and separate in a normal-pressure column the organic phase to obtain the product with a yield of 78%. The yield is the percentage between the weight of the product and the calculated theoretical output calculated according to OH[UCC]AZMB.

### (24) Synthesis of DMTr[GG]OH

Add DMTr[GG]AZMB (7.55g, 4.81mmol) obtained in step (9) and Ph₃P (5.04g, 19.2mmol) into a 500ml round bottom flask, add 240ml of dioxane/water (v:v=9:1) to dissolve the sample, stir and react at room temperature for 8h, end the reaction, remove he solvent and conduct fast liquid chromatography by a high performance separation, purification and preparation chromatograph (model: Combiflash Companion/TS; manufacturer: Teledyne ISCO INC.) to obtain the crude product containing triphenylphosphine oxide and the removed 3'-protective group, dry it and keep it for future use.

### (25) Synthesis of DMTr[GG]PO⁻

Add 1,2,4-triazole (1.50g, 22.1mmol) and the double distilled triethylamine (3.58g, 35.4mmol) into a 100ml round bottom flask, add 16.6ml of anhydrous CH₂Cl₂ to dissolve it, dropwise add 2-chlorophenyl dichlorophosphate (2.17g, 8.8mmol) dissolved in 8.8ml of anhydrous CH₂Cl₂ under the cooling condition of ice bath and stir and react 1h. Change the ice bath into ice salt bath, adjust temperature to -10□~5□, dropwise add product DMTr[GG]OH obtained in step (24) dissolved in 13.3ml of anhydrous CH₂Cl₂, and stir and react 4h in ice salt bath. Add 20ml of 1M TEAB, continue the stirring and reaction 0.5h. stop the reaction, separate the solution to obtain an organic phase, wash it with 1M TEAB three times, dry the organic phase over anhydrous Na₂SO₄ and filter, concentrate and separate in a normal-pressure column the organic phase to obtain the product with a yield of 60%. The yield is the percentage between the weight of the product and the calculated theoretical output calculated according to DMTr[GG]AZMB.

### (26) Synthesis of DMTr[UCA]OH

A method same as step (24) is adopted to synthesize DMTr[UCA]OH, except that DMTr[UCA]AZMB (11.3g, 4.69mmol) obtained in step (11) and Ph₃P (4.96g, 18.7mmol) are adopted.

### (27) Synthesis of DMTr[UCA]PO⁻

A method same as step (25) is adopted to prepare DMTr[UCA]PO⁻, except that 19.5ml of anhydrous CH₂Cl₂ is used to dissolve 1,2,4-triazole (1.77g, 26.0mmol) and the double distilled triethylamine (4.21g, 41.7mmol), 10.5ml of anhydrous CH₂Cl₂ is used to dissolve 2-chlorophenyl dichlorophosphate (2.56g, 10.4mmol) and 15.6ml of anhydrous CH₂Cl₂ is used to dissolve DMTr[UCA]OH obtained in step (26). The yield is 79%. The yield is the percentage between the weight of the product and the calculated theoretical output calculated according to DMTr[UCA]AZMB.

### (28) Synthesis of DMTr[UGC]OH

A method same as step (24) is adopted to synthesize DMTr[UGC]OH, except that DMTr[UGC]AZMB (10.6g, 4.42mmol) obtained in step (10) and Ph₃P (4.63g, 17.7mmol) are adopted.

### (29) Synthesis of DMTr[UGC]PO⁻

A method same as step (25) is adopted to prepare DMTr[UGC]PO⁻, except that 19.4ml of anhydrous CH₂Cl₂ is used to dissolve 1,2,4-triazole (1.76g, 25.9mmol) and the double distilled triethylamine (4.18g, 41.4mmol), 10.4ml of anhydrous CH₂Cl₂ is used to dissolve 2-chlorophenyl dichlorophosphate (2.54g, 10.3mmol) and 15.5ml of anhydrous CH₂Cl₂ is used to dissolve DMTr[UGC]OH obtained in step (28). The yield is 71%. The yield is the percentage between the weight of the product and the calculated theoretical output calculated according to DMTr[UGC]AZMB.

### (30) Synthesis of DMTr[GUU]OH

A method same as step (24) is adopted to synthesize DMTr[GUU]OH, except that DMTr[GUU]AZMB (5.41g, 2.36mmol) obtained in step (13) and Ph₃P (2.47g, 9.43mmol) are adopted and the use amount of dioxane/water (v:v=9:1) is 120ml.

### (31) Synthesis of DMTr[GUU]PO⁻

A method same as step (25) is adopted to prepare DMTr[GUU]PO⁻, except that 9.8ml of anhydrous CH₂Cl₂ is used to dissolve 1,2,4-triazole (891mg, 13.1mmol) and the double distilled triethylamine (2.12g, 21.0mmol), 5.2ml of anhydrous CH₂Cl₂ is used to dissolve 2-chlorophenyl dichlorophosphate (2.54g, 10.3mmol) and 7.9ml of anhydrous CH₂Cl₂ is used to dissolve DMTr[GUU]OH obtained in step (30). The yield is 64%. The yield is the percentage between the weight of the product and the calculated theoretical output calculated according to DMTr[GUU]AZMB.

### (32) Synthesis of DMTr[GUG]OH

A method same as step (24) is adopted to synthesize DMTr[GUG]OH, except that DMTr[GUG]AZMB (4.40g, 1.83mmol) obtained in step (14) and Ph₃P (1.92g, 7.33mmol) are adopted and the use amount of dioxane/water (v:v=9:1) is 900ml.

### (33) Synthesis of DMTr[GUG]PO⁻

A method same as step (25) is adopted to prepare DMTr[GUG]PO-, except that 6.8ml of anhydrous CH₂Cl₂ is used to dissolve 1,2,4-triazole (622mg, 9.15mmol) and the double distilled triethylamine (1.48g, 14.7mmol), 3.6ml of anhydrous CH₂Cl₂ is used to dissolve 2-chlorophenyl dichlorophosphate (898mg, 3.66mmol) and 5.5ml of anhydrous CH₂Cl₂ is used to dissolve DMTr[GUG]OH obtained in step (32). The yield is 64%. The yield is the percentage between the weight of the product and the calculated theoretical output calculated according to DMTr[GUG]AZMB.

### (34) Synthesis of DMTr[CGCA]OH

A method same as step (24) is adopted to synthesize DMTr[CGCA]OH, except that DMTr[CGCA]AZMB (4.77g, 1.67mmol) obtained in step (22) and Ph₃P(1.74g, 6.66mmol) are adopted.

### (35) Synthesis of DMTr[CGCA]PO⁻

A method same as step (25) is adopted to prepare DMTr[CGCA]PO⁻, except that 10.0ml of anhydrous CH₂Cl₂ is used to dissolve 1,2,4-triazole (604mg, 8.88mmol) and the double distilled triethylamine (1.62g, 16.0mmol), 3.5ml of anhydrous CH₂Cl₂ is used to dissolve 2-chlorophenyl dichlorophosphate (873mg, 3.55mmol) and 5.4ml of anhydrous CH₂Cl₂ is used to dissolve DMTr[CGCA] OH obtained in step (34). The yield is 64%. The yield is the percentage between the weight of the product and the calculated theoretical output calculated according to DMTr[GUU]AZMB.

### (36) Synthesis of DMTr[UUCC]OH

A method same as step (24) is adopted to synthesize DMTr[UUCC]OH, except that DMTr[UUCC]AZMB (3.85g, 1.19memo1) obtained in step (23) and Ph₃P (1.25g, 4.77mmol) are adopted and the use amount of dioxane/water (v:v=9:1) mixed solution is 60ml.

### (37) Synthesis of DMTr[UUCC]PO⁻

A method same as step (25) is adopted to prepare DMTr[UUCC]PO⁻, except that 9.8ml of anhydrous CH₂Cl₂ is used to dissolve 1,2,4-triazole (891mg, 13.1mol) and the double distilled triethylamine (2.12g, 21.0mmol), 5.2ml of anhydrous CH₂Cl₂ is used to dissolve 2-chlorophenyl dichlorophosphate (1.29g, 5.25mmol) and 5.5ml of anhydrous CH₂Cl₂ is used to dissolve DMTr[UUCC]OH obtained in step (36). The yield is 86%. The yield is the percentage between the weight of the product and the calculated theoretical output calculated according to DMTr[UUCC]AZMB.

### (38) Synthesis of DMTr[GGAACC]AZMB

Add DMTr[GG]PO⁻ (2.74g, 2.15mmol) obtained in step (25) and HO[AACC]AZMB (2.70g, 1.05mmol) obtained in step (21) into a 100ml round bottom flask, add 10ml of anhydrous pyridine to fully dissolve them, add MSNT (0.873g, 2.94mmol) by batch, react at room temperature for 3h, add 1ml of 1M TEAB, stir 25min, stop the reaction, pour the solution into about 100ml of CH₂Cl₂, wash it with 1M TEAB three times, separate the solution to obtain an organic phase, dry the organic phase over anhydrous Na₂SO₄ and filter, concentrate and separate in a normal-pressure column the organic phase to obtain the product with a yield of 48%.

### (39) Synthesis of DMTr[UCACA]AZMB

A method same as step (38) is adopted to synthesize DMTr[UCACA]AZMB, except that DMTr[UCA]PO-(4.84g, 2.16mmol) obtained in step (27) and HO[CA]AZMB (2.01g, 1.57mmol) obtained in step (5) are adopted, the use amount of anhydrous pyridine is 15ml and MSNT (1.31g, 4.40mmol). The yield is 66%.

### (40) Synthesis of DMTr[UCAACG]AZMB

A method same as step (38) is adopted to synthesize DMTr[UCAACG]AZMB, except that DMTr[UCA]PO⁻ (4.56g, 2.03mmol) obtained in step (27) and HO[ACG]AZMB (2.70g, 1.41mmol) obtained in step (20) are adopted, the use amount of anhydrous pyridine is 15ml and MSNT (1.17g, 3.95mmol). The yield is 60%.

### (41) Synthesis of DMTr[CGCAUU]AZMB

A method same as step (38) is adopted to synthesize DMTr[CGCAUU]AZMB, except that DMTr[CGCA]PO⁻ (3.2g, 1.07mmol) obtained in step (35) and HO[UU]AZMB (1.05g, 1.00mmol) obtained in step (4) are adopted, the use amount of anhydrous pyridine is 10ml and MSNT (832mg, 2.80mmol). The yield is 87%.

### (42) Synthesis of DMTr[UGCGC]AZMB

A method same as step (38) is adopted to synthesize DMTr[UGCGC]AZMB, except that DMTr[UGC]PO⁻ (7.00g, 3.14mmol) obtained in step (29) and HO[GC]AZMB (3.10g, 2.46mmol) obtained in step (7) are adopted, the use amount of anhydrous pyridine is 25ml and MSNT (2.05g, 6.89mmol). The yield is 78%.

### (43) Synthesis of DMTr[GUUGAU]AZMB

A method same as step (38) is adopted to synthesize DMTr[GUUGAU]AZMB, except that DMTr[GUU]PO⁻ (3.20g, 1.51mmol) obtained in step (31) and HO[GAU]AZMB (2.30g, 1.27mmol) obtained in step (19) are adopted, the use amount of anhydrous pyridine is 12ml and MSNT (1.06g, 3.56mmol). The yield is 74%.

### (44) Synthesis of DMTr[GUGAGG]AZMB

A method same as step (38) is adopted to synthesize DMTr[GUGAGG]AZMB, except that DMTr[GUG]PO⁻ (2.6g, 1.16mmol) obtained in step (33) and HO[AGG]AZMB (1.90g, 0.99mmol) obtained in step (18) are adopted, the use amount of anhydrous pyridine is 10ml and MSNT(832mg, 2.8mmol). The yield is 74%.

### (45) Synthesis of DMTr[UUCCUU]AZMB

A method same as step (38) is adopted to synthesize DMTr[UUCCUU]AZMB, except that DMTr[UUCC]PO⁻ (2.80g, 1.02mmol) obtained in step (37) and HO[UU]AZMB (0.94g, 0.90mmol) obtained in step (4) are adopted, the use amount of anhydrous pyridine is 10ml and MSNT (748mg, 2.52mmol). The yield is 82%.

### (46) Synthesis of HO[UCACA]AZMB

Dissolve DMTr[UCACA]AZMB (3.80g, 1.03mmol) obtained in step (39) in 15ml of CH₂Cl₂, slowly add 6% CF₃COOH, ensure the concentration of CF₃COOH in the system is 4% and stir at room temperature 30min. Add saturated NaHCO₃ to neutralize the solution, separate the solution to obtain an organic phase, wash the organic phase with 15ml of saturated NaHCO₃ once, dry the organic phase over anhydrous Na₂SO₄ and filter, concentrate and separate in a normal-pressure column the organic phase to obtain the product with a yield of 84%.

### (47) Synthesis of HO[CGCAUU]AZMB

A method same as step (46) is adopted to prepare HO[CGCAUU]AZMB, except that DMTr[CGCAUU]AZMB (3.8g, 0.97mmol) obtained in step (41) is adopted. The yield is 58%.

### (48) Synthesis of HO[GUUGAU]AZMB

A method same as step (46) is adopted to prepare HO[GUUGAU]AZMB, except that DMTr[GUUGAU]AZMB (3.50g, 0.94mmol) obtained in step (43) is adopted. The yield is 58%.

### (49) Synthesis of HO[UUCCUU]AZMB

A method same as step (46) is adopted to prepare HO[UUCCUU]AZMB, except that DMTr[UUCCUU]AZMB (2.90g, 0.79mmol) obtained in step (45) is adopted. The yield is 87%.

### (50) Synthesis of DMTr[GGAACC]PO⁻

Add DMTr[GGAACC]AZMB (2.20g, 0.50mmol) obtained in step (38) and Ph₃P (524mg, 2.00mmol) into a 100ml round bottom flask, add 25ml of dioxane/water (v:v=9:1), stir and react at room temperature 18h, remove the solvent through rotary evaporation and conduct fast liquid chromatography by a high performance separation, purification and preparation chromatograph (model: Combiflash Companion/TS; manufacturer: Teledyne ISCO INC.) to obtain 2.00g of the crude product containing triphenylphosphine oxide and the removed 3'-protective group, dry it and keep it for future use.

Add 1,2,4-triazole (170mg, 2.50mmol) and the double distilled triethylamine (454mg, 4.50mmol) into a 100ml round bottom flask, add 1.5ml of anhydrous CH₂Cl₂ to dissolve them, dropwise add 2-chlorophenyl dichlorophosphate (246mg, 1.0mmol) dissolved in 1.0ml of anhydrous CH₂Cl₂ under the cooling condition of ice bath and stir and react 1h. Change the ice bath into ice salt bath, adjust temperature to -10°C~-5°C, dropwise add product DMTr[GGAACC]OH obtained in the above step, and stir and react 4h. Add 10ml of 1M TEAB and continue the stirring and reaction 0.5h. Stop the reaction, separate the solution to obtain an organic phase, wash it with 1M TEAB three times, dry the organic phase over anhydrous Na₂SO₄ and filter, concentrate and separate in a normal-pressure column the organic phase to obtain the product with 54% of total yield in the two steps.

### (51) Synthesis of DMTr[UCAACG]PO⁻

A method same as step (50) is adopted to synthesize DMTr[UCAACG]PO⁻, except that DMTr[UCAACG]AZMB (3.60g, 0.94mmol) obtained in step (40) is adopted. The yield in the two steps is 66%.

### (52) Synthesis of DMTr[UGCGC]PO⁻

A method same as step (50) is adopted to synthesize DMTr[UGCGC]PO⁻, except that DMTr[UGCGC]AZMB (6.40g, 1.90mmol) obtained in step (42) is adopted. The yield in the two steps is 70%.

### (53) Synthesis of DMTr[GUGAGG]PO⁻

A method same as step (50) is adopted to synthesize DMTr[GUGAGG]PO⁻, except that DMTr[GGAACC]AZMB (3.00g, 0.75mmol) obtained in step (38) is adopted. The yield in the two steps is 41 %.

### (54) Synthesis of DMTr[GGAACCUCACA]AZMB

Add DMTr[GGAACC]PO⁻ (1.2g, 0.26mmol) obtained in step (50) and HO[UCACA]AZMB (0.85g, 0.25mmol) obtained in step (46) into a 50ml round bottom flask, add 5ml of anhydrous pyridine to fully dissolve them, add MSNT (208mg, 0.70mmol) by batch and react at room temperature 8h. Add 1ml of 1M TEAB, stir 25min, stop the reaction, pour the reaction solution into about 50ml of CH₂Cl₂, wash it with 1M TEAB three times, separate the solution to obtain an organic phase, dry the organic phase over anhydrous Na₂SO₄ and filter, concentrate and separate in a normal-pressure column the organic phase to obtain 1.40g of the product with a yield of 71%.

### (55) Synthesis of DMTr[UGCGCGUUGAU]AZMB

Add DMTr[UGCGC]PO⁻ (2.52g, 0.72mmol) obtained in step (52) and HO[GUUGAU]AZMB (2.5g, 0.71mmol) obtained in step (486) into a 50ml round bottom flask, add 8ml of anhydrous pyridine to fully dissolve them, add MSNT (500mg, 1.68mmol) by batch and react at room temperature 8h. Add 1ml of 1M TEAB, stir 25min, stop the reaction, pour the reaction solution into about 50ml of CH₂Cl₂, wash it with 1M TEAB three times, separate the solution to obtain an organic phase, dry the organic phase over anhydrous Na₂SO₄ and filter, concentrate and separate in a normal-pressure column the organic phase to obtain the product with a yield of 49%.

### (56) Synthesis of DMTr[UCAACGCGCAUU]AZMB

Add DMTr[UCAACG]PO-(2.43g, 0.61mmol) obtained in step (51) and HO[CGCAUU]AZMB (1.85g, 0.51mmol) obtained in step (47) into a 50ml round bottom flask, add 8ml of anhydrous pyridine to fully dissolve them, add MSNT (424mg, 1.43mmol) by batch and react at room temperature 8h. Add 1ml of 1M TEAB, stir 25min, stop the reaction, pour the reaction solution into about 50ml of CH₂Cl₂, wash it with 1M TEAB three times, separate the solution to obtain an organic phase, dry the organic phase over anhydrous Na₂SO₄, filter, concentrate and separate in a normal-pressure column the organic phase to obtain the product and directly use the product in next reaction.

### (57) Synthesis of DMTr[GUGAGGUUCCUU]AZMB

Add DMTr[GUGAGG]PO⁻ (1.40g, 0.30mmol) obtained in step (53) and HO[UUCCUU]AZMB (1.24g, 0.37mmol) obtained in step (49) into a 50ml round bottom flask, add 6ml of anhydrous pyridine to fully dissolve them, add MSNT (249mg, 0.84mmol) by batch and react at room temperature 8h. Add 1ml of 1M TEAB, stir 25min, stop the reaction, pour the reaction solution into about 50ml of CH₂Cl₂, wash it with 1M TEAB three times, separate the solution to obtain an organic phase, dry the organic phase over anhydrous Na₂SO₄, filter, concentrate and separate in a normal-pressure column the organic phase to obtain the product and directly use the product in next reaction.

### (58) Synthesis of HO[UCAACGCGCAUU]AZMB

Dissolve DMTr[UCAACGCGCAUU]AZMB obtained in step (56) in 10ml of CH₂Cl₂, slowly add 6% CF₃COOH, ensure the concentration of CF₃COOH in the system is 4% and stir at room temperature 30min. Add saturated NaHCO₃ to neutralize the solution, separate the solution to obtain an organic phase, wash the organic phase with saturated NaHCO₃ once, dry the organic phase over anhydrous Na₂SO₄ and filter, concentrate and separate in a normal-pressure column the organic phase to obtain the product with a yield of 53%. The yield is the percentage between the weight of the product and the calculated theoretical output calculated according to HO[CGCAUU]AZMB.

### (59) Synthesis of HO[GUGAGGUUCCUU]AZMB

Dissolve DMTr[GUGAGGUUCCUU]AZMB obtained in step (57) in 10ml of CH₂Cl₂, slowly add 6% CF₃COOH, ensure the concentration of CF₃COOH in the system is 4% and stir at room temperature 30min. Add saturated NaHCO₃ to neutralize the solution, separate the solution to obtain an organic phase, wash the organic phase with saturated NaHCO₃ once, dry the organic phase over anhydrous Na₂SO₄ and filter, concentrate and separate in a normal-pressure column the organic phase to obtain the product with a yield of 54%. The yield is the percentage between the weight of the product and the calculated theoretical output calculated according to HO[UUCCUU]AZMB.

### (60) Synthesis of DMTr[GGAACCUCACA]PO⁻

Add DMTr[GGAACCUCACA]AZMB (1.40g, 0.18mmol) obtained in step (54) and Ph₃P (200mg, 0.76mmol) into a 100ml round bottom flask, add 10ml of dioxane/water (v:v=9:1) to dissolve them, stir and react at room temperature 28h, remove the solvent through rotary evaporation, conduct fast liquid chromatography by a high performance separation, purification and preparation chromatograph to obtain 1.2g of crude product containing triphenylphosphine oxide and the removed 3'-protective group, dry it and keep it for future use.

Add 1,2,4-triazole (65mg, 0.96mmol) and the double distilled triethylamine (162mg, 1.60mmol) into a 25ml round bottom flask, add 0.5ml of anhydrous CH₂Cl₂ to dissolve them, dropwise add 2-chlorophenyl dichlorophosphate (98mg, 0.40mmol) dissolved in 0.5ml of anhydrous CH₂Cl₂ under the cooling condition of ice bath and stir and react 1h. Change the ice bath into ice salt bath, adjust temperature to -10°C~-5°C, dropwise add product DMTr[GGAACCUCACA]OH obtained in the above step, and stir and react 5.5h. Add 3ml of 1M TEAB and continue the stirring and reaction 1h. Separate the solution to obtain an organic phase, wash it with 1M TEAB three times, dry the organic phase over anhydrous Na₂SO₄ and filter, concentrate and separate in a normal-pressure column the organic phase to obtain the product with a yield of 43%. The yield is the percentage between the weight of the product and the calculated theoretical output calculated according to DMTr[GGAACCUCACA]AZMB.

### (61) Synthesis of DMTr[UGCGCGUUGAU]PO⁻

Add DMTr[UGCGCGUUGAU]AZMB (1.95g, 0.28mmol) obtained in step (55) and Ph₃P (350mg, 1.34mmol) into a 100ml round bottom flask, add 10ml of dioxane/water (v:v=9:1) to dissolve the sample, stir and react at room temperature 28h, end the reaction, remove the solvent through rotary evaporation, conduct fast liquid chromatography by a high performance separation, purification and preparation chromatograph to obtain 1.45g of crude product containing triphenylphosphine oxide and the removed 3'-protective group, dry it and keep it for future use.

Add 1,2,4-triazole (90mg, 1.32mmol) and the double distilled triethylamine (224mg, 2.22mmol) into a 100ml round bottom flask, add 1.0ml of anhydrous CH₂Cl₂ to dissolve the sample, dropwise add 2-chlorophenyl dichlorophosphate (137mg, 0.56mmol) dissolved in 0.5ml of anhydrous CH₂Cl₂ under the cooling condition of ice bath and stir and react 1h. Change the ice bath into ice salt bath, adjust temperature to -10°C~-5°C, dropwise add product DMTr[UGCGCGUUGAU]OH obtained in the above step and dissolved in anhydrous CH₂Cl₂, and stir and react 5.5h. Add 3ml of 1M TEAB and continue the stirring and reaction 1h. Stop the reaction, separate the solution to obtain an organic phase, wash it with 1M TEAB three times, dry the organic phase over anhydrous Na₂SO₄ and filter, concentrate and separate in a normal-pressure column the organic phase to obtain the product with a yield of 34.4%. The yield is the percentage between the weight of the product and the calculated theoretical output calculated according to DMTr[UGCGCGUUGAU]AZMB.

### (62) Synthesis of DMTr[GGAACCUCACAUCAACGCGCAUU]AZMB

Add DMTr[GGAACCUCACA]PO⁻ (560mg, 70mmol) obtained in step (60) and HO[UCAACGCGCAUU]AZMB (500mg, 70mmol) obtained in step (58) into a 25ml round bottom flask, add 1.5ml of anhydrous pyridine to fully dissolve the sample, add MSNT (62mg, 0.21mmol) by batch and react at room temperature 8h. Add 1ml of 1M TEAB, stir 25min, stop the reaction, pour the reaction solution into about 50ml of CH₂Cl₂, wash it with 1M TEAB three times, separate the solution to obtain an organic phase, dry the organic phase over anhydrous Na₂SO₄ and filter, concentrate and separate in a normal-pressure column the organic phase to obtain the product with a yield of 96%.

### (63) Synthesis of DMTr[UGCGCGUUGAUGUGAGGUUCCUU]AZMB

Add DMTr[UGCGCGUUGAU]PO⁻ (560mg, 79umol) obtained in step (61) and HO[GUGAGGUUCCUU]AZMB (600mg, 79umol) obtained in step (59) into a 25ml round bottom flask, add 1.5ml of anhydrous pyridine to fully dissolve the sample, add MSNT (70mg, 0.24mmol) by batch and react at room temperature 8h. Add 1ml of 1M TEAB, stir 25min, stop the reaction, pour the reaction solution into about 50ml of CH₂Cl₂, wash it with 1M TEAB three times, separate the solution to obtain an organic phase, dry the organic phase over anhydrous Na₂SO₄ and filter, concentrate and separate in a normal-pressure column the organic phase to obtain the product with a yield of 88%.

### Example 4

This example synthesizes oligonucleotides.
Synthesis target: 5'- GGAACCUCACAUCAACGCGCAUU -3'
And 5'- UGCGCGUUGAUGUGAGGUUCCUU -3'

The protective groups are removed by a method same as that in Example 2, except that DMTr[GGAACCUCACAUCAACGCGCAUU]AZMB and DMTr[UGCGCGUUGAUGUGAG GUUCCUU]AZMB substitutes DMTr[CGAAAGAACG]AZMB synthesized in Example 3 are used, respectively.

As a result of sequencing, the prepared sequences are consistent with the designed sequences.

## Claims

1. A method for preparing oligonucleotide, including reacting the compound represented by Formula (1) with the compound represented by Formula (2) in a liquid reaction medium under the condition of condensation reaction to obtain the compound represented by Formula (3), wherein, said condition of condensation reaction includes the use of 1-(mesitylene-2-sulfonyl)-3-nitro-1,2,4-triazole (MSNT) as condensing agent, 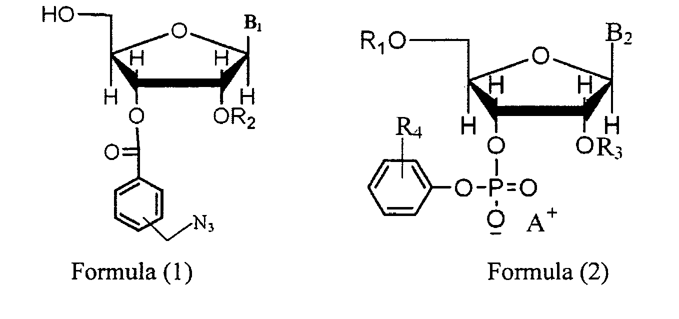 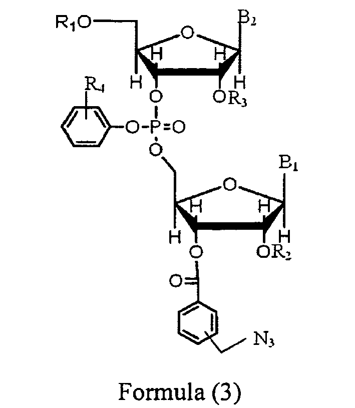 Wherein:
R₁ represents 4,4'-dimethoxytriphenylmethyl, RNA or DNA;
R₂ and R₃ independently represent a sterically hindered silane protective group;
R₄ represents a halogen atom;
A⁺ represents a tri-alkyl ammonium ion;
B₁ and B₂ independently represent guanyl substituted with N-acyl, adeninyl substituted with N-acyl, cytosine substituted with N-acyl, thymine or uracil.

2. The method as in claim 1, wherein the condition of the condensation reaction further includes: the reaction medium is pyridine ; relative to 1mol of the compound represented by Formula (1), the amount of the compound represented by Formula (2) is 0.8-3mol, the amount of the condensing agent is 2-5mol, and the amount of the reaction medium is 5-50L; the reaction temperature is 10-50°C and the reaction time is 0.5-10h.

3. The method as in claim 1, wherein R₂ and R₃ independently represent tert-butyl dimethyl silyl, phenyl dimethyl silyl, tert-butyl diphenyl silyl or triisopropyl silyl; R₄ represents Cl or Br; each of the alkyl groups in the tri-alkyl ammonium ion has 1-6 carbon atoms; the acyl is benzoyl, isobutyryl or acetyl.

4. The method as in claim 1, wherein in Formula (1) or (3), -CH₂-N₃ is at ortho-position; in Formula (2) or (3), R₄ is at ortho-position.

5. The method as in claim 1, wherein the compound represented by Formula (1) is prepared by reacting the compound represented by Formula (4) with the compound represented by Formula (5) under the condition of condensation reaction and then removing 5'-protective group, 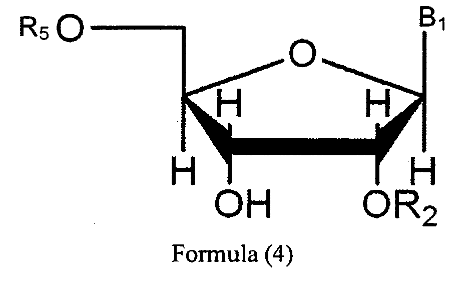 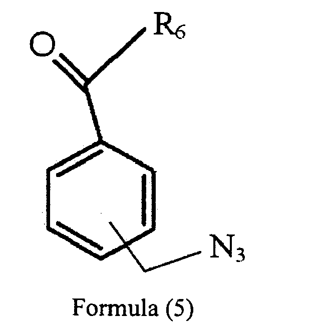 Where:
R₂ represents a sterically hindered silane protective group;
R₅ represents 4,4'-dimethoxytriphenylmethyl;
R₆ represents a halogen atom;
B₁ represents guanyl substituted with N-acyl, adeninyl substituted with N-acyl, cytosine substituted with N-acyl, thymine or uracil.

6. The method as in claim 5, wherein the condition of the condensation reaction includes: N-methyl imidazole is used as an adjuvant; one or more of dichloromethane and pyridine is used as reaction medium; relative to 1mol of the compound represented by Formula (4), the amount of the compound represented by Formula (5) is 1-5mol, the amount of the adjuvant is 1.8-3.5mol, and the amount of the reaction medium is 20-200L; the reaction temperature is -10°C~10°C and the reaction time is 5-100h.

7. The method as in claim 5 or 6, wherein the process for preparing the compound represented by Formula (5) includes the following steps:
(1) in the presence of benzoyl peroxide, the compound represented by Formula (9) reacts with N-halogenated succinimide to obtain the compound represented by Formula (10);
(2) the compound represented by Formula (10) reacts with alkali metal azide to obtain the compound represented by Formula (11);
(3) the compound represented by Formula (11) is hydrolyzed and acylated to obtain the compound represented by Formula (5), 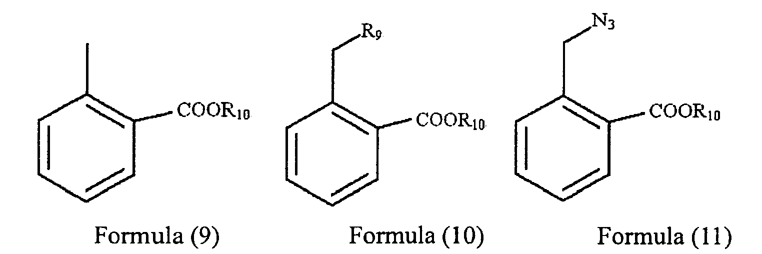 Where: R₉ represents a halogen atom; R₁₀ represents C₁-C₄ alkyl.

8. The method as in claim 7, wherein in step (1), the reaction medium is one or more of carbon tetrachloride, chloroform, benzene, toluene and heptane ; relative to 1mol of the compound represented by Formula (9), the amount of N- halogenated succinimide is 1-3mol, the amount of benzoyl peroxide is 0.01-0.1mol, the amount of the reaction medium is 5-20L ; the reaction temperature is 80~120°C and the reaction time is 0.5-6h;
in step (2), the reaction medium is one or more of ethanol, acetone, N,N-dimethyl formamide and dimethyl sulfoxide ; relative to 1mol of the compound represented by Formula (10), the amount of alkali metal azide is 1-3mol, the amount of the reaction medium is 3-10L; the reaction temperature is 0°C~80°Cand the reaction time is 2-30h;
in step (3), the hydrolysis includes the reaction between the compound represented by Formula (11) and the alcohol-water mixed solution of alkali metal hydroxide wherein relative to 1mol of the compound represented by Formula (10), the amount of alkali metal hydroxide is 5-100mol, the reaction temperature is 0°C~50°C, the reaction time is 0.1-2h and the concentration of alkali metal hydroxide in the alcohol-water mixed solution is 5-10wt%.

9. The method as in claim 1, wherein the compound represented by Formula (2) is obtained through the reaction of the compound represented by Formula (6), the compound represented by Formula (7) and trialkyl amine in a reaction medium in the presence of a catalyst, the catalyst being one or more of 1,2,4-triazole, triethylamine and pyridine, the reaction medium being one or more of dichloromethane, dioxane and tetrahydrofuran, 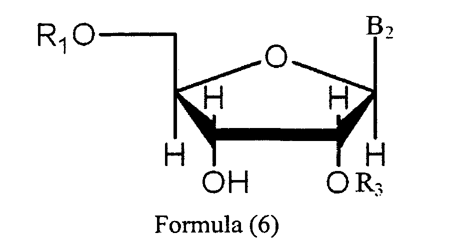 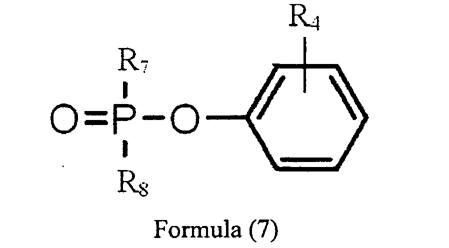 Where:
R₁ represents 4,4'-dimethoxytriphenylmethyl, RNA or DNA;
R₃ represents a sterically hindered silane protective group;
R₄, R₇ and R₈ independently represent a halogen atom;
B₂ represents guanyl substituted with N-acyl, adeninyl substituted with N-acyl, cytosine substituted with N-acyl, thymine or uracil.

10. The method as in claim 9, wherein the temperature of the reaction is -10°C~10°C and the reaction time is 0.5-10h; relative to 1mol of the compound represented by Formula (6), the amount of the compound represented by Formula (7) is 1-5mol, the amount of trialkyl amine is 1-50mol and the amount of catalyst is 2-10mol.

## Patentansprüche

1. Verfahren zur Herstellung eines Oligonukleotids, einschließlich Umsetzen der durch die Formel (1) repräsentierten Verbindung mit der durch die Formel (2) repräsentierten Verbindung in einem flüssigen Reaktionsmedium unter der Bedingung einer Kondensationsreaktion unter Erhalt der durch die Formel (3) repräsentierten Verbindung, wobei die Bedingung einer Kondensationsreaktion die Verwendung von 1-(Mesitylen-2-sulfonyl)-3-nitro-1,2,4-triazol (MSNT) als Kondensationsmittel beinhaltet, 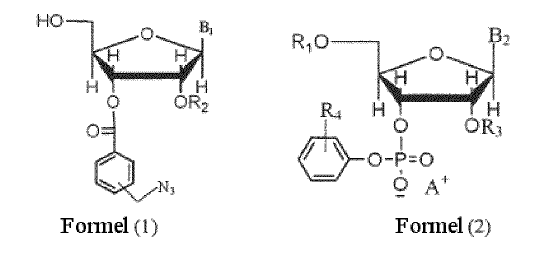 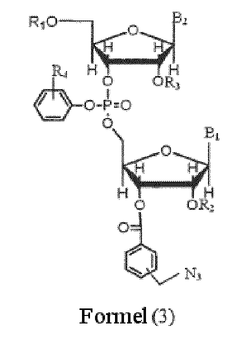 wobei:
R₁ für 4,4'-Dimethoxytriphenylmethyl, RNA oder DNA steht;
R₂ und R₃ unabhängig für eine sterisch gehinderte Silanschutzgruppe stehen;
R₄ für ein Halogenatom steht;
A⁺ für ein Trialkylammoniumion steht;
B₁ und B₂ unabhängig für mit N-Acyl substituiertes Guanyl, mit N-Acyl substituiertes Adeninyl, mit N-Acyl substituiertes Cytosin, Thymin oder Uracil stehen.

2. Verfahren nach Anspruch 1, wobei die Bedingung der Kondensationsreaktion ferner Folgendes beinhaltet: bei dem Reaktionsmedium handelt es sich um Pyridin; relativ zu 1 mol der durch die Formel (1) repräsentierten Verbindung beträgt die Menge der durch die Formel (2) repräsentierten Verbindung 0,8-3 mol, die Menge des Kondensationsmittels 2-5 mol und die Menge des Reaktionsmediums 5-50 l; die Reaktionstemperatur liegt bei 10-50°C und die Reaktionszeit bei 0,5-10 h.

3. Verfahren nach Anspruch 1, wobei R₂ und R₃ unabhängig für tert.-Butyldimethylsilyl, Phenyldimethylsilyl, tert.-Butyldiphenylsilyl oder Triisopropylsilyl stehen, R₄ für Cl oder Br steht, die Alkylgruppen in dem Trialkylammoniumion jeweils 1-6 Kohlenstoffatome aufweisen, es sich bei dem Acyl um Benzoyl, Isobutyryl oder Acetyl handelt.

4. Verfahren nach Anspruch 1, wobei in der Formel (1) oder (3) -CH₂-N₃ in ortho-Stellung vorliegt, in der Formel (2) oder (3) R₄ in ortho-Stellung vorliegt.

5. Verfahren nach Anspruch 1, wobei die durch die Formel (1) repräsentierte Verbindung hergestellt wird, indem man die durch die Formel (4) repräsentierte Verbindung mit der durch die Formel (5) repräsentierten Verbindung unter der Bedingung einer Kondensationsreaktion umsetzt und danach die 5'-Schutzgruppe entfernt, 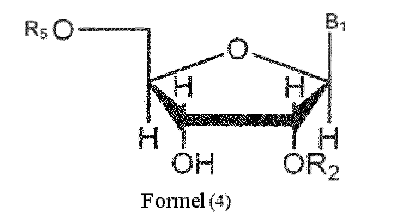 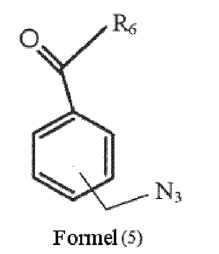 worin:
R₂ für eine sterisch gehinderte Silanschutzgruppe steht;
R₅ für 4 , 4 '-Dimethoxytriphenylmethyl steht;
R₆ für ein Halogenatom steht;
B₁ für mit N-Acyl substituiertes Guanyl, mit N-Acyl substituiertes Adeninyl, mit N-Acyl substituiertes Cytosin, Thymin oder Uracil steht.

6. Verfahren nach Anspruch 5, wobei die Bedingung der Kondensationsreaktion Folgendes beinhaltet: N-Methylimidazol wird als Adjuvans verwendet; Dichlormethan oder/und Pyridin wird als Reaktionsmedium verwendet; relativ zu 1 mol der durch die Formel (4) repräsentierten Verbindung beträgt die Menge der durch die Formel (5) repräsentierten Verbindung 1-5 mol, die Menge des Adjuvans 1,8-3,5 mol und die Menge des Reaktionsmediums 20-200 l; die Reaktionstemperatur liegt bei -10~10°C und die Reaktionszeit bei 5-100 h.

7. Verfahren nach Anspruch 5 oder 6, wobei der Vorgang zur Herstellung der durch die Formel (5) repräsentierten Verbindung die folgenden Schritte beinhaltet:
(1) in Gegenwart von Benzoylperoxid reagiert die durch die Formel (9) repräsentierte Verbindung mit N-halogeniertem Succinimid unter Erhalt der durch die Formel (10) repräsentierten Verbindung;
(2) die durch die Formel (10) repräsentierte Verbindung reagiert mit Alkaliazid unter Erhalt der durch die Formel (11) repräsentierten Verbindung;
(3) die durch die Formel (11) repräsentierte Verbindung wird unter Erhalt der durch die Formel (5) repräsentierten Verbindung hydrolysiert und acyliert, 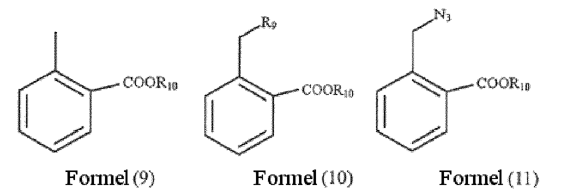 worin: R₉ für ein Halogenatom steht; R₁₀ für C₁-C₄-Alkyl steht.

8. Verfahren nach Anspruch 7, wobei es sich bei dem Reaktionsmedium in Schritt (1) um eines oder mehrere aus der Gruppe Tetrachlorkohlenstoff, Chloroform, Benzol, Toluol und Heptan handelt; relativ zu 1 mol der durch die Formel (9) repräsentierten Verbindung die Menge an N-halogeniertem Succinimid 1-3 mol, die Menge an Benzoylperoxid 0,01-0,1 mol, die Menge des Reaktionsmediums 5-20 1 beträgt; die Reaktionstemperatur bei 80-120°C und die Reaktionszeit bei 0,5-6 h liegt;
es sich bei dem Reaktionsmedium in Schritt (2) um eines oder mehrere aus der Gruppe Ethanol, Aceton, N,N-Dimethylformamid und Dimethylsulfoxid handelt; relativ zu 1 mol der durch die Formel (10) repräsentierten Verbindung die Menge an Alkaliazid 1-3 mol, die Menge des Reaktionsmediums 3-10 1 beträgt; die Reaktionstemperatur bei 0-80°C und die Reaktionszeit bei 2-30 h liegt;
die Hydrolyse in Schritt (3) die Reaktion zwischen der durch die Formel (11) repräsentierten Verbindung und der Alkohol-Wasser-Mischlösung von Alkalihydroxid beinhaltet, wobei relativ zu 1 mol der durch die Formel (10) repräsentierten Verbindung die Menge an Alkalihydroxid 5-100 mol beträgt, die Reaktionstemperatur bei 0°C-50°C liegt, die Reaktionszeit bei 0,1-2 h liegt und die Konzentration an Alkalihydroxid in der Alkohol-Wasser-Mischlösung 5-10 Gew.-% beträgt.

9. Verfahren nach Anspruch 1, wobei man die durch die Formel (2) repräsentierte Verbindung über die Umsetzung der durch die Formel (6) repräsentierten Verbindung, der durch die Formel (7) repräsentierten Verbindung und Trialkylamin in einem Reaktionsmedium in Gegenwart eines Katalysators erhält, wobei es sich bei dem Katalysator um einen oder mehrere aus der Gruppe 1,2,4-Triazol, Triethylamin und Pyridin und bei dem Reaktionsmedium um eines oder mehrere aus der Gruppe Dichlormethan, Dioxan und Tetrahydrofuran handelt, 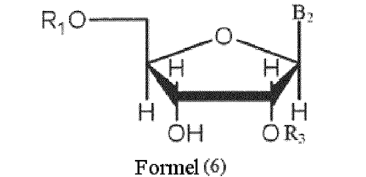 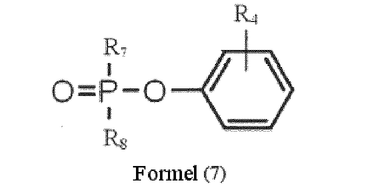 worin:
R₁ für 4,4'-Dimethoxytriphenylmethyl, RNA oder DNA steht;
R₃ für eine sterisch gehinderte Silanschutzgruppe steht;
R₄, R₇ und R₈ unabhängig für ein Halogenatom stehen;
B₂ für mit N-Acyl substituiertes Guanyl, mit N-Acyl substituiertes Adeninyl, mit N-Acyl substituiertes Cytosin, Thymin oder Uracil steht.

10. Verfahren nach Anspruch 9, wobei die Temperatur der Reaktion bei -10°C~10°C und die Reaktionszeit bei 0,5-10 h liegt; relativ zu 1 mol der durch die Formel (6) repräsentierten Verbindung die Menge der durch die Formel (7) repräsentierten Verbindung 1-5 mol, die Menge an Trialkylamin 1-50 mol und die Menge an Katalysator 2-10 mol beträgt.

## Revendications

1. Procédé pour préparer un oligonucléotide, comprenant la réaction du composé représenté par la formule (1) avec le composé représenté par la formule (2) dans un milieu de réaction liquide dans les conditions de réaction de condensation pour obtenir le composé représenté par la formule (3), dans lequel, lesdites conditions de réaction de condensation comprennent l'utilisation de 1-(mésitylène-2-sulfonyl)-3-nitro-1,2,4-triazole (MSNT) en tant qu'agent de condensation, 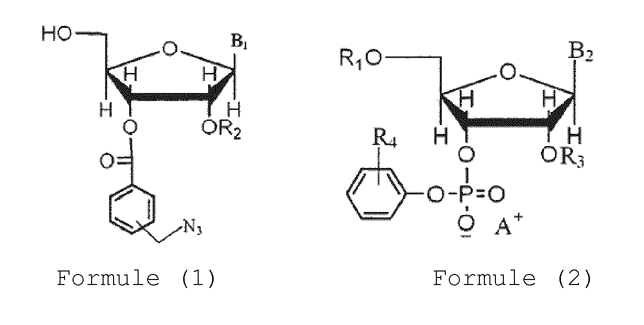 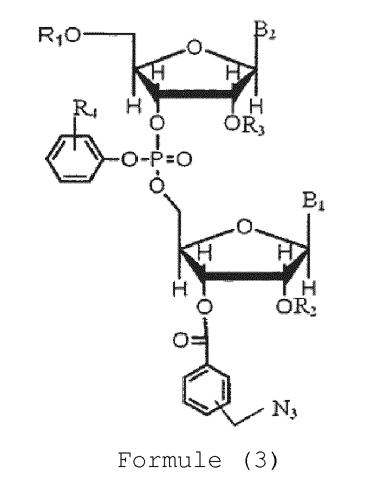 où :
R₁ représente 4,4'-diméthoxytriphénylméthyle, un ARN ou un ADN ;
R₂ e t R₃ représentent indépendamment un groupe protecteur de silane stériquement encombré ;
R₄ représente un atome d'halogène ;
A⁺ représente un ion trialkylammonium ;
B₁ et B₂ représentent indépendamment guanyle substitué par N-acyle, adéninyle substitué par N-acyle, cytosine substituée par N-acyle, thymine ou uracile.

2. Procédé selon la revendication 1, dans lequel les conditions de la réaction de condensation comprennent en outre : le milieu de réaction est de la pyridine ; par rapport à 1 mole du composé représenté par la formule (1), la quantité du composé représenté par la formule (2) est de 0,8 à 3 moles, la quantité de l'agent de condensation est de 2 à 5 moles, et la quantité du milieu de réaction est de 5 à 50 1 ; la température de réaction est de 10 à 50 °C et le temps de réaction est de 0,5 à 10 h.

3. Procédé selon la revendication 1, dans lequel R₂ e t R₃ représentent indépendamment tert-butyldiméthylsilyle, phényldiméthylsilyle, tert-butyldiphénylsilyle ou triisopropylsilyle ; R₄ représente Cl ou Br ; chacun des groupes alkyle dans l'ion trialkylammonium a de 1 à 6 atomes de carbone ; l'acyle est benzoyle, isobutyryle ou acétyle.

4. Procédé selon la revendication 1, dans lequel dans la formule (1) ou (3), -CH₂-N₃ est à la position ortho ; dans la formule (2) ou (3), R₄ est à la position ortho.

5. Procédé selon la revendication 1, dans lequel le composé représenté par la formule (1) est préparé par réaction du composé représenté par la formule (4) avec le composé représenté par la formule (5) dans les conditions de réaction de condensation et ensuite élimination du groupe protecteur 5', 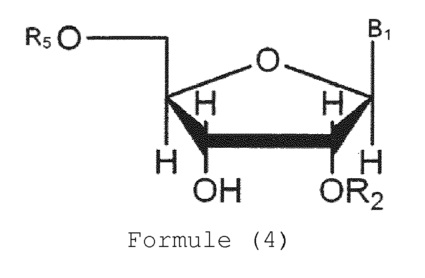 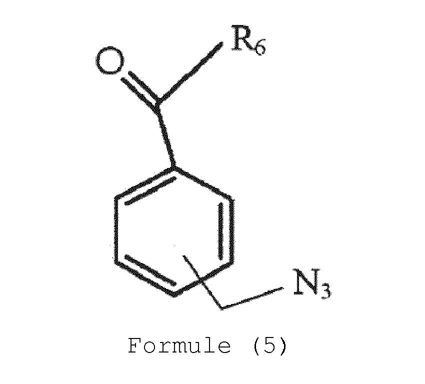 où :
R₂ représente un groupe protecteur de silane stériquement encombré ;
R₅ représente 4,4'-diméthoxytriphénylméthyle ;
R₆ représente un atome d'halogène ;
B₁ représente guanyle substitué par N-acyle, adéninyle substitué par N-acyle, cytosine substituée par N-acyle, thymine ou uracile.

6. Procédé selon la revendication 5, dans lequel les conditions de la réaction de condensation comprennent : N-méthylimidazole est utilisé en tant qu'adjuvant ; un ou plusieurs du dichlorométhane et de la pyridine est utilisé en tant que milieu de réaction ; par rapport à 1 mole du composé représenté par la formule (4), la quantité du composé représenté par la formule (5) est de 1 à 5 moles, la quantité de l'adjuvant est de 1, 8 à 3, 5 moles, et la quantité du milieu de réaction est de 20 à 200 1 ; la température de réaction est de -10 ° C à 10 0 °C et le temps de réaction est de 5 à 100 h.

7. Procédé selon la revendication 5 ou 6, dans lequel le procédé pour préparer le composé représenté par la formule (5) comprend les étapes suivantes :
(1) en présence de peroxyde de benzoyle, le composé représenté par la formule (9) réagit avec le succinimide N-halogéné pour obtenir le composé représenté par la formule (10) ;
(2) le composé représenté par la formule (10) réagit avec un azide de métal alcalin pour obtenir le composé représenté par la formule (11) ;
(3) le composé représenté par la formule (11) est hydrolysé et acylé pour obtenir le composé représenté par la formule (5), 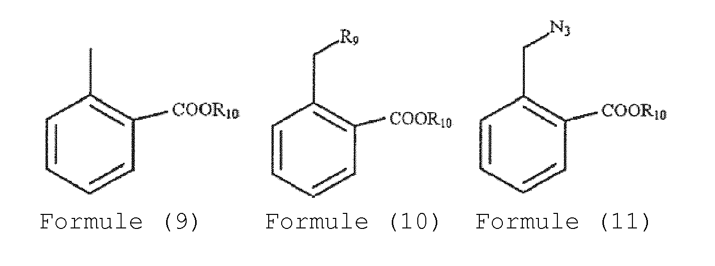 où : R₉ représente un atome d'halogène ; R₁₀ représente alkyle en C₁-C₄.

8. Procédé selon la revendication 7, dans lequel, dans l'étape (1), le milieu de réaction est l'un ou plusieurs parmi le tétrachlorure de carbone, le chloroforme, le benzène, le toluène et l'heptane ; par rapport à 1 mole du composé représenté par la formule (9), la quantité de succinimide N-halogéné est de 1 à 3 moles, la quantité de peroxyde de benzoyle est de 0,01 1 à 0,1 mole, la quantité du milieu de réaction est de 5 à 20 1 ; la température de réaction est de 80 à 120 °C et le temps de réaction est de 0,5 à 6 h ; dans l'étape (2), le milieu de réaction est l'un ou plusieurs parmi l'éthanol, l'acétone, le N,N-diméthylformamide et le diméthylsulfoxyde ; par rapport à 1 mole du composé représenté par la formule (10), la quantité d'azide de métal alcalin est de 1 à 3 moles, la quantité du milieu de réaction est de 3 à 10 1 ; la température de réaction est de 0 °C à 80 °C et le temps de réaction est de 2 à 30 h ;
dans l'étape (3), l'hydrolyse comprend la réaction entre le composé représenté par la formule (11) et la solution mixte d'alcool-eau d'hydroxyde de métal alcalin, où, par rapport à 1 mole du composé représenté par la formule (10), la quantité d'hydroxyde de métal alcalin est de 5 à 100 mole, la température de réaction est de 0 °C à 50 °C, le temps de réaction est de 0,1 à 2 h et la concentration d'hydroxyde de métal alcalin dans la solution mixte d'alcool-eau est de 5 à 10 % en poids.

9. Procédé selon la revendication 1, dans lequel le composé représenté par la formule (2) est obtenu par la réaction du composé représenté par la formule (6), le composé représenté par la formule (7) et la trialkylamine dans un milieu de réaction en présence d'un catalyseur, le catalyseur étant un ou plusieurs parmi le 1,2,4-triazole, la triéthylamine et la pyridine, le milieu de réaction étant l'un ou plusieurs parmi le dichlorométhane, le dioxane et le tétrahydrofurane, 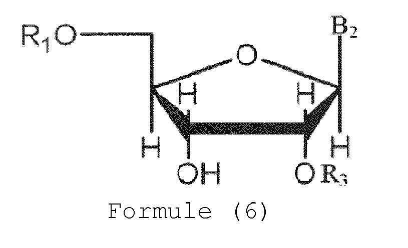 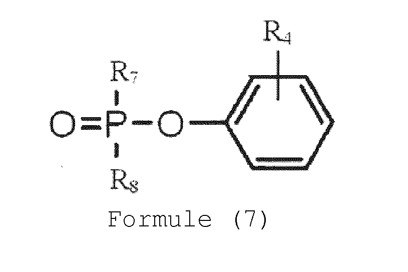 où:
R₁ représente 4,4'-diméthoxytriphénylméthyle, un ARN ou un ADN ;
R₃ représente un groupe protecteur de silane stériquement encombré ;
R₄, R₇ et R₈ représentent indépendamment un atome d'halogène ;
B₂ représente guanyle substitué par N-acyle, adéninyle substitué par N-acyle, cytosine substituée par N-acyle, thymine ou uracile.

10. Procédé selon la revendication 9, dans lequel la température de la réaction est de -10 °C à 10 °C et le temps de réaction est de 0,5 à 10 h ; par rapport à 1 mole du composé représenté par la formule (6), la quantité du composé représenté par la formule (7) est de 1 à 5 mole, la quantité de trialkylamine est de 1 à 50 moles et la quantité de catalyseur est de 2 à 10 moles.
